# EUROPEAN PATENT SPECIFICATION

(11) **EP 3 194 368 B1**
(45) Date of publication and mention of the grant of the patent: **23.12.2020**
(21) Application number: 15843004.1
(22) Date of filing: 17.09.2015
(51) Int. Cl.: C07D 221/06, C07D 417/04, C07D 413/04, C07D 209/48, A61K 31/473, A61K 31/404, A61P 35/00, A61P 43/00

(54) **BENZO-HETEROCYCLIC COMPOUNDS AND THEIR APPLICATIONS**
BENZO-HETEROCYCLISCHE VERBINDUNGEN UND DEREN ANWENDUNGEN
COMPOSÉS BENZO-HÉTÉROCYCLIQUES ET LEURS APPLICATIONS

(30) Priority: 19.09.2014 US 201462052617 P
(43) Date of publication of application: 26.07.2017
(73) Proprietor: MacKay Medical Foundation the Presbyterian Church in Taiwan MacKay Memorial Hospital, Taipei City 10449 (TW)
(72) Inventor: SHIH, Tzenge-Lien, New Taipei City 251 (TW); CHANG, Chia-Ming, Taipei City 112 (TW); WEI, Tsai-Yin, Changhua County 512 (TW); LIU, Jen-Wei, New Taipei City 236 (TW); LU, Yen-Ta, Wenshan District Taipei City 116 (TW)
(74) Representative: Lavoix
(86) International application number: PCT/CN2015/089865
(87) International publication number: WO 2016/041511

(56) References cited:
- WO-A1-2008/084496
- WO-A1-2008/084496
- WO-A2-00/32577
- WO-A2-2008/155594
- CN-A- 1 857 221
- CN-A- 102 206 203
- CN-A- 102 206 203
- CN-A- 102 731 493
- CN-A- 102 731 493
- BUU-HOI N P ET AL: "PHTALONIMIDES (1,3,4-TRIOXO-1,2,3,4-TETRAHYDROISOQUINOLI NES) OF POTENTIAL BIOLOGICAL INTEREST", JOURNAL OF HETEROCYCLIC CHEMISTRY, vol. 5, no. 4, 1 January 1968 (1968-01-01) , pages 545-547, XP000906859, WILEY-BLACKWELL PUBLISHING, INC, US ISSN: 0022-152X, DOI: 10.1002/JHET.5570050416
- MCKENNEY, RYAN K. ET AL: "The heterocyclic ring of 4-substituted-1,8-naphthalimides is NOT inert to nucleophilic attack, contrary to earlier reports", ORGANIC & BIOMOLECULAR CHEMISTRY, vol. 11, no. 26, 1 January 2013 (2013-01-01), pages 4390-4396, XP002777511, ISSN: 1477-0520, DOI: 10.1039/C3OB40639C
- MIAO L ET AL: "A TDDFT and PCM-TDDFT studies on absorption spectra of N-substituted 1,8-naphthalimides dyes", JOURNAL OF MOLECULAR STRUCTURE (THEOCHEM), vol. 865, no. 1-3, 30 September 2008 (2008-09-30), pages 79-87, XP024522482, ELSEVIER SCIENCE PUBLISHERS B.V., AMSTERDAM, NL ISSN: 0166-1280, DOI: 10.1016/J.THEOCHEM.2008.06.029 [retrieved on 2008-07-09]
- ZHENGNENG JIN ET AL: "Synthesis and fluorescence property of some novel 1,8-naphthalimide derivatives containing a thiophene ring at the C-4 position", DYES AND PIGMENTS, vol. 96, no. 1, 1 August 2012 (2012-08-01) , pages 204-210, XP028942548, ISSN: 0143-7208, DOI: 10.1016/J.DYEPIG.2012.07.018
- AIBIN WU ET AL: "Novel Naphthalimide-Benzoic Acid Conjugates as Potential Apoptosis-Inducing Agents: Design, Synthesis, and Biological Activity : Preapoptotic Naphthalimide Conjugates", CHEMICAL BIOLOGY & DRUG DESIGN., vol. 78, no. 6, 29 September 2011 (2011-09-29), pages 941-947, XP055419616, GB ISSN: 1747-0277, DOI: 10.1111/j.1747-0285.2011.01232.x
- AIBIN WU ET AL: "Novel naphthalimide derivatives as potential apoptosis-inducing agents: Design, synthesis and biological evaluation", EUROPEAN JOURNAL OF MEDICINAL CHEMISTRY, vol. 44, no. 11, 16 July 2009 (2009-07-16) , pages 4674-4680, XP055419614, FR ISSN: 0223-5234, DOI: 10.1016/j.ejmech.2009.07.011
- SETHI KALYAN K ET AL: "Carbonic anhydrase inhibitors: Synthesis and inhibition of the cytosolic mammalian carbonic anhydrase isoforms I, II and VII with benzene sulfonamides incorporating 4,5,6,7-tetrachlorophthalimide moiety", BIOORGANIC & MEDICINAL CHEMISTRY, vol. 21, no. 17, 26 June 2013 (2013-06-26) , pages 5168-5174, XP028690157, ISSN: 0968-0896, DOI: 10.1016/J.BMC.2013.06.035
- SETHI KALYAN K ET AL: "Carbonic anhydrase inhibitors: Synthesis and inhibition of the human carbonic anhydrase isoforms I, II, VII, IX and XII with benzene sulfonamides incorporating 4,5,6,7-tetrabromophthalimide moiety", BIOORGANIC & MEDICINAL CHEMISTRY, vol. 21, no. 19, 2 August 2013 (2013-08-02), pages 5973-5982, XP028706279, ISSN: 0968-0896, DOI: 10.1016/J.BMC.2013.07.044
- SETHI KALYAN K ET AL: "Carbonic anhydrase inhibitors: Synthesis and inhibition of the human carbonic anhydrase isoforms I, II, IX and XII with benzene sulfonamides incorporating 4- and 3-nitrophthalimide moieties", BIOORGANIC & MEDICINAL CHEMISTRY, vol. 22, no. 5, 31 January 2014 (2014-01-31), pages 1586-1595, XP028616700, ISSN: 0968-0896, DOI: 10.1016/J.BMC.2014.01.031
- C. DAVID ANDERSSON ET AL: "Discovery of Ligands for ADP-Ribosyltransferases via Docking-Based Virtual Screening", JOURNAL OF MEDICINAL CHEMISTRY, vol. 55, no. 17, 12 July 2012 (2012-07-12) , pages 7706-7718, XP055419618, ISSN: 0022-2623, DOI: 10.1021/jm300746d
- ABDEL-AZIZ ALAA A-M ET AL: "Carbonic anhydrase inhibitory activity of sulfonamides and carboxylic acids incorporating cyclic imide scaffolds", BIOORGANIC & MEDICINAL CHEMISTRY LETTERS, vol. 24, no. 22, 2 October 2014 (2014-10-02), pages 5185-5189, XP029088724, PERGAMON, AMSTERDAM, NL ISSN: 0960-894X, DOI: 10.1016/J.BMCL.2014.09.076
- ALAA A.-M. ABDEL-AZIZ ET AL.: 'Carbonic anhydrase inhibitory activity of sulfonamides and carboxylic acids incorporating cyclic imide scaffolds.' BIOORGANIC & MEDICINAL CHEMISTRY LETTERS vol. 24, no. 22, 02 October 2014, pages 5185 - 5189, XP029088724
- AIBIN WU ET AL.: 'Novel naphthalimide derivatives as potential apoptosis-inducing agents: Design, synthesis and biological evaluation.' EUROPEAN JOURNAL OF MEDICINAL CHEMISTRY vol. 44, 16 July 2009, pages 4674 - 4680, XP055419614
- AIBIN WU ET AL.: 'Novel naphthalimide-benzoic acid conjugates as potential apoptosis-inducing agents: design, synthesis, and biological activity.' CHEMICAL BIOLOGY & DRUG DESIGN. vol. 78, no. 6, 22 August 2011, pages 941 - 947, XP055419616
- SETHI, KALYAN K. ET AL.: 'Carbonic anhydrase inhibitors: Synthesis and inhibition of the human carbonic anhydrase isoforms I, II, VII, IX and XII with benzene sulfonamides incorporating 4, 5, 6, 7-tetrabromophthalimide moiety.' BIOORGANIC & MEDICINAL CHEMISTRY. vol. 21, no. 19, 02 August 2013, pages 5973 - 5982, XP028706279
- SETHI, KALYAN K. ET AL.: 'Carbonic anhydrase inhibitors: Synthesis and inhibition of the human carbonic anhydrase isoforms I, II, IX and XII with benzene sulfonamides incorporating 4- and 3-nitrophthalimide moieties.' BIOORGANIC & MEDICINAL CHEMISTRY. vol. 22, no. 5, 31 January 2014, pages 1586 - 1595, XP028616700
- ANDERSSON, C. DAVID ET AL.: 'Discovery of Ligands for ADP-Ribosyltransferases via Docking- Based Virtual Screening.' JOURNAL OF MEDICINAL CHEMISTRY vol. 55, no. 17, 23 July 2012, pages 7706 - 7718, XP055419618

## Description

### Field of the Invention

The present disclosure relates to compounds for treatment or prophylaxis of sepsis or septic shock and cancers. More particularly, the present invention relates to benzo-heterocyclic derivatives and the applications thereof in inhibiting PD-L1 expression and increasing HLA-DR expression.

### Background of the Invention

Sepsis is defined as Systemic Inflammatory Response Syndrome (SIRS) resulting from microbial invasion that may originate anywhere in the body, such as genitourinary tract, liver or biliary tract, gastrointestinal tract, lungs, surgical wounds, etc. The infection is usually confirmed by a positive blood culture, and may lead to shock, termed septic shock, which is a consequence of sepsis that leads to vital organ failure. Clinically, sepsis is defined by both the infection and SIRS. Infection is suspected when an above-normal white blood cell (WBC) count is noted, or by a chest radiograph consistent with pneumonia. Evidence of SIRS includes derangement of vital signs.

Currently, sepsis represents a major clinical problem with limited treatment options. Only one drug has been approved by FDA for the treatment of severe sepsis, a recombinant human activated protein C (Xigris®). However, significant side effects (e.g., serious bleeding) have been reported in a significant number of patients. Further, Xigris® has no effect on severe sepsis patients with an Acute Physiology and Chronic Health Evaluation (APACHE) II score of ≤ 25 (Abraham E et al., N Engl J Med (2005) 353, 1332-1341.).

Sepsis has recently been reported to associate with a form of immunosuppression called "immunoparalysis" characterized in a decrease in the expression of human leukocyte antigen (HLA) DR (HLA-DR) and increase in the expression of programmed death-ligand 1 (PD-L1) in monocytes (Hotchkiss RS and Opal S, (2010) N Engl J Med 363, 87-89*).* Thus, if an agent were successful in reverting the unique expression of HLA-DR and PD-L1 associated with sepsis patients, such agent may be useful for the treatment or prophylaxis of sepsis or septic shock.

On the other hand, clinically, up-regulation of PD-L1 on monocytes/APCs has been documented in a variety of human chronic infectious diseases such as HIV, viral hepatitis, cytomegalovirus infection and latent mycobacterium tuberculosis (Saresella M, Rainone V, Al-Daghri NM, Clerici M, Trabattoni D (2012) The PD-1/PD-L1 pathway in human pathology. Curr Mol Med 12: 259-267*),* leading to inhibition of T cell effector function recognized as "T-cell exhaustion"(Watanabe T, Bertoletti A, Tanoto TA (2010) PD-1/PD-L1 pathway and T-cell exhaustion in chronic hepatitis virus infection. J Viral Hepat 17: 453-458*).* Similar immune impairment with an increase in PD-L1/PD-1 interaction has also been observed in patients with different forms and stages of cancers (Chen IH, Lai YL, Wu CL, Chang YF, Chu CC, et al. (2010) Immune impairment in patients with terminal cancers: influence of cancer treatments and cytomegalovirus infection. Cancer Immunol Immunother 59: 323-334*).* Recently, there are 3 immunotherapeutic drugs, two anti-PD-1 mAbs (Keytruda, MDS; Nivolumab, BMS) and one anti-CTLA-4 mAb (Yervoy, BMS) marketed for the treatment of cancers. All 3 biologics are currently approved for the treatment of melanoma; however trials are actively conducted on a variety of cancers including lung cancers, reno-urinary cancers, breast cancers and other solid tumors. Early results from several global trials hail surprising results not only with clinical efficacy but also showing durable effects (Topalian SL, Hodi FS, Brahmer JR, Gettinger SN, Smith DC, et al. (2012) Safety, activity, and immune correlates of anti-PD-1 antibody in cancer. N Engl J Med 366: 2443-2454*.;* Brahmer JR, Tykodi SS, Chow LQ, Hwu WJ, Topalian SL, et al. (2012) Safety and activity of anti-PD-L1 antibody in patients with advanced cancer. N Engl J Med 366: 2455-2465*).* Therefore, cancer immunotherapy has been projected to feature in more than 60% of all cancer treatment plans.

CN 102 731 493; CN 102 206 203; BUU-HOI N P et al (JOURNAL OF HETEROCYCLIC CHEMISTRY, vol. 5, no. 4, 1968 pages 545-547); MCKENNEY et al (Organic and Biomolecular Chemistry, 11(26), 4390-4396, 2013); MIAO et al (JOURNAL OF MOLECULAR STRUCTURE (THEOCHEM), vol. 865, no. 1-3, 2008, 79-87); ZHENGNENG JIN et al (DYES AND PIGMENTS, vol. 96, no. 1 , 2012, 204-210); AIBIN WU et al (CHEMICAL BIOLOGY & DRUG DESIGN, vol. 78, no. 6, 2011, 941-947,); WO 2008/084496; WO 00132577; AIBIN WU et al (EUROPEAN JOURNAL OF MEDICINAL CHEMISTRY, vol. 44, no. 11, 2009, 4674-4680); SEïHI KALYAN K et al (BIOORGANIC & MEDICINAL CHEMISTRY, vol. 21, no. 17, 2013, 5168-5174); SETHI KALYAN K et al (BIOORGANIC & MEDICINAL CHEMISTRY, vol. 21, no. 19, 2013, 5973-5982); SETHI KALYAN K et al (BIOORGANIC & MEDICINAL CHEMISTRY, vol.22, no.5, 2014, pages 1586-1595); C. DAVID ANDERSSON et al (JOURNAL OF MEDICINAL CHEMISTRY,vol. 55, no. 17, 2012, 7706-7718); ABDEL-AZIZ ALAA A-M et al (BIOORGANIC & MEDICINAL CHEMISTRY LETTERS, vol. 24, no. 22, 2014, 5185-5189) disclose 1,8 naphtalimides or phtalimides or derivatives thereof.

In view of the foregoing, there exists in this art a need of an agent that may reverse characteristic expression of HLA-DR and PD-L1 associated with sepsis patients. Such agent may be useful as a lead compound for the manufacture of a medicament for treating patients having or suspected of having sepsis or septic shock and cancers.

### Summary of the Invention

The invention concerns a compound having the following Formula (I), 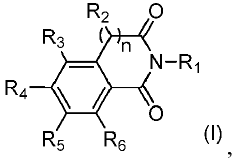 wherein the compound is selected from the group consisted of the following:

| Compound Name | n | R₁ | R₂ | R₃ | R₄ | R₅ | R₆ |
|---|---|---|---|---|---|---|---|
| ML-A1-B | 1 | -CH₂CH₂N(CH₃)₂ | =CH-CH=CH- | | 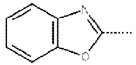 | H | H |

or a tautomer, stereoisomer or enantiomer thereof, or a solvate, or a pharmaceutically acceptable salt thereof.

The following compounds are also disclosed:

| Compound Name | n | R₁ | R₂ | R₃ | R₄ | R₅ | R₆ |
|---|---|---|---|---|---|---|---|
| ML-A1-K | 1 | -CH₂CH₂OH | =CH-CH=CH- | | 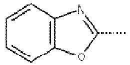 | H | H |
| ML-A1-I | 1 | -(CH₂)₃CH₃ | =CH-CH=CH- | | 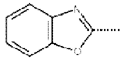 | H | H |
| ML-C19-B | 1 | 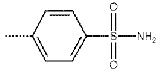 | =CH-CH=CH- | | Br | H | H |
| ML-C19-PH2 | 0 | 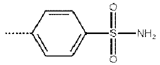 | - | H | H | Br | H |
| ML-C19-PH4 | 0 | 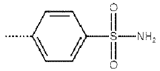 | - | H | H | CH₃ | H |
| ML-C19-PH6 | 0 | 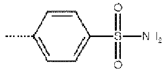 | - | H | H | H | NO₂ |

According to an embodiment of the invention, the compound is 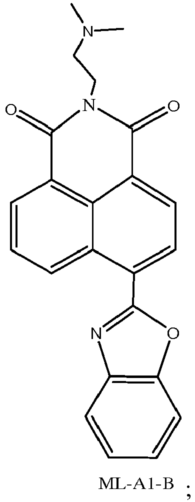 or a tautomer, a stereoisomer, an enantiomer, or a solvate thereof, or a pharmaceutically acceptable salt thereof.

It is also disclosed a compound of Formula (I), 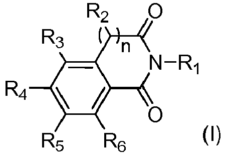 wherein
n is 0 or 1,
R₁ is selected from halogen, C₁₋₁₀alkyl, C₂₋₁₀alkenyl, C₂₋₁₀alkynyl, haloC₁₋₁₀alkyl, hydroxyC₁₋₁₀alkyl, aminoC₁₋₁₀alkyl, C₃₋₁₀cycloalkyl, 3 to 10 membered cyclic heterocycloalkyl having 1 to 3 heteroatoms selected from N, S and O, C₆₋₁₀aryl or 5 to 10 membered mono- or bi-cyclic heteroaryl having from 1 to 3 heteroatoms selected from N, S and O, wherein the amino moiety of aminoalkyl is unsubstituted or substituted by one or two alkyl groups, and the cycloalkyl, heterocycloalkyl, aryl and heteroaryl are substituted by -SO₂NH₂, or -CONH₂;
R₂ is selected from hydrogen, halogen, hydroxy, amino, cyano, nitro, C₁₋₁₀alkyl, C₂₋₁₀alkenyl, C₂₋₁₀alkynyl, C₁₋₁₀alkoxy, C₁₋₁₀alkylthio, C₁₋₁₀alkylamino, haloC₁₋₁₀alkyl, hydroxyC₁₋₁₀alkyl, aminoC₁₋₁₀alkyl, C₃₋₁₀cycloalkyl, 3 to 10 membered cyclic heterocycloalkyl having 1 to 3 heteroatoms selected from N, S and O, C₆₋₁₀aryl or 5 to 10 membered mono- or bi-cyclic heteroaryl having from 1 to 3 heteroatoms selected from N, S and O;
R₃ is selected from hydrogen, halogen, hydroxy, amino, cyano, nitro, C₁₋₁₀alkyl, C₂₋₁₀alkenyl, C₂₋₁₀alkynyl, C₁₋₁₀alkoxy, C₁₋₁₀alkylthio, C₁₋₁₀alkylamino, haloC₁₋₁₀alkyl, hydroxyC₁₋₁₀alkyl, aminoC₁₋₁₀alkyl, C₃₋₁₀cycloalkyl, 3 to 10 membered cyclic heterocycloalkyl having 1 to 3 heteroatoms selected from N, S and O, C₆₋₁₀aryl or 5 to 10 membered mono- or bi-cyclic heteroaryl having from 1 to 3 heteroatoms selected from N, S and O; or
R₂ and R₃ together with carbon atoms to which they attach form a benzene ring, wherein the benzene ring is unsubstituted or substituted by one or more groups independently selected from halogen, hydroxy, amino, cyano, nitro, C₁₋₁₀alkyl, C₂₋₁₀alkenyl, C₂₋₁₀alkynyl, C₁₋₁₀alkoxy, C₁₋₁₀alkylthio, C₁₋₁₀alkylamino, haloC₁₋₁₀alkyl, hydroxyC₁₋₁₀alkyl, aminoC₁₋₁₀alkyl, C₃₋₁₀cycloalkyl, 3 to 10 membered cyclic heterocycloalkyl having 1 to 3 heteroatoms selected from N, S and O, C₆₋₁₀aryl or 5 to 10 membered mono- or bi-cyclic heteroaryl having from 1 to 3 heteroatoms selected from N, S and O, with the proviso that n is 1; and
R₄, R₅ and R₆ are each independently selected from hydrogen, halogen, amino, hydroxy, cyano, nitro, C₁₋₁₀alkyl, C₂₋₁₀alkenyl, C₂₋₁₀alkynyl, C₁₋₁₀alkoxy, C₁₋₁₀alkylthio, C₁₋₁₀alkylamino, haloC₁₋₁₀alkyl, hydroxyC₁₋₁₀alkyl, aminoC₁₋₁₀alkyl, C₃₋₁₀cycloalkyl, 3 to 10 membered cyclic heterocycloalkyl having 1 to 3 heteroatoms selected from N, S and O, C₆₋₁₀aryl or 5 to 10 membered mono- or bi-cyclic heteroaryl having from 1 to 3 heteroatoms selected from N, S and O; wherein the cycloalkyl, heterocycloalkyl, aryl and heteroaryl in R₂ to R₆ may be unsubstituted or substituted by one to four groups selected from halogen, hydroxy, amino, cyano, nitro, C₁₋₁₀alkyl, C₂₋₁₀alkenyl, C₂₋₁₀alkynyl, C₁₋₁₀alkoxy, C₁₋₁₀alkylthio, C₁₋₁₀alkylamino, haloC₁₋₁₀alkyl, hydroxyC₁₋₁₀alkyl, aminoC₁₋₁₀alkyl, C₃₋₁₀cycloalkyl;
with provisos that (1) when n is 1, R₁ is 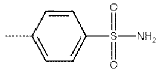 and R₂ and R₃ together with carbon atoms to which they attach form a benzene ring, R₄, R₅ and R₆ cannot simultaneously represent hydrogen; (2) when n is 0 and R₁ is selected from the cycloalkyl, heterocycloalkyl, aryl or heteroaryl, R₃, R₄, R₅ and R₆ are not simultaneously hydrogen; (3) when n is 0, R₁ is 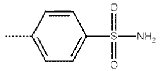 and R₃ and R₆ simultaneously represent hydrogen, if one of R₄ and R₅ is hydrogen, the other cannot be methyl, tert-butyl or nitro; (4) when n is 0, R₁ is 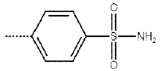 and R₃ and R₆ simultaneously represent hydrogen, R₄ and R₅ cannot simultaneously represent chloro; and (5) when n is 0 and R₁ is 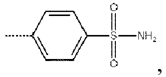 R₃, R₄, R₅ and R₆ cannot simultaneously represent chloro
or a tautomer, stereoisomer or enantiomer thereof, or a solvate, prodrug or a pharmaceutically acceptable salt thereof.

In some embodiments of the disclosed Formula (I), n is 1; R₁ is (C₁₋₁₀(di)alkylamino)C₁₋₁₀alkyl, hydroxyC₁₋₁₀alkyl or C₆₋₁₀aryl substituted by -SO₂NH₂ or -CO₂NH₂; R₂ and R₃ together with carbon atoms to which they attach form a benzene ring, wherein the benzene ring is unsubstituted or substituted by one or more groups independently selected from halogen, amino, cyano, nitro or C₁₋₁₀alkyl; R₄ is H, halogen or 5- to 10-membered mono- or bi-cyclic, unsubstituted or substituted heteroaryl having from 1 to 3 heteroatoms selected from N, S and O; and R₅ and R₆ are each independently hydrogen, halogen, amino, cyano, nitro, C₁₋₁₀alkyl or haloC₁₋₁₀alkyl. In some embodiments of the disclosed Formula (I), n is 0, R₁ is C₆₋₁₀aryl substituted by -SO₂NH₂ or -CO₂NH₂; and R₃, R₄, R₅ and R₆ are each independently selected from hydrogen, halogen, amino, cyano, nitro or C₁₋₁₀alkyl.

The invention also provides a pharmaceutical composition for use in treating or preventing a PD-L1 associated cancer in a subject by inhibiting PD-L1, wherein the pharmaceutical composition comprises the compound having the following formula (I): 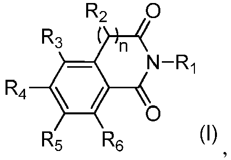 wherein the compound is selected from the group consisted of the following:

| Compound Name | n | R₁ | R₂ | R₃ | R₄ | R₅ | R₆ |
|---|---|---|---|---|---|---|---|
| ML-A1-B | 1 | -CH₂CH₂N(CH₃)₂ | =CH-CH=CH- | | 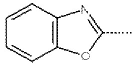 | H | H |
| ML-A1-C | 1 | -CH₂CH₂N(CH₃)₂ | =CH-CH=CH- | | 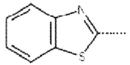 | H | H |
| ML-C19-A | 1 | 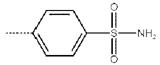 | =CH-CH=CH- | | H | H | H |

or a tautomer, stereoisomer or enantiomer thereof, or a solvate, or a pharmaceutically acceptable salt thereof.

It is also disclosed a pharmaceutical composition for use in treating or preventing a PD-L1 associated cancer in a subject by inhibiting PD-L1, wherein the pharmaceutical composition comprises the compound having the following formula (I): 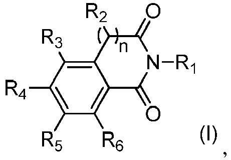 wherein the compound is selected from the group consisted of the following:

| Compound Name | n | R₁ | R₂ | R₃ | R₄ | R₅ | R₆ |
|---|---|---|---|---|---|---|---|
| ML-A1-K | 1 | -CH₂CH₂OH | =CH-CH=CH- | | 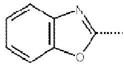 | H | H |
| ML-A1-L | 1 | -CH₂CH₂OH | =CH-CH=CH- | | 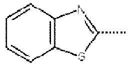 | H | H |
| ML-A1-I | 1 | -(CH₂)₃CH₃ | =CH-CH=CH- | | 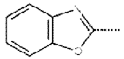 | H | H |

| ML-C19-B | 1 | 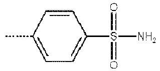 | =CH-CH=CH- | | Br | H | H |
|---|---|---|---|---|---|---|---|
| ML-C19-PH2 | 0 | 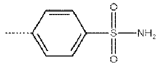 | -- | H | H | Br | H |
| ML-C19-PH3 | 0 | 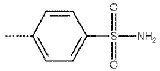 | -- | Br | Br | Br | Br |
| ML-C19-PH4 | 0 | 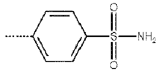 | -- | H | H | CH₃ | H |
| ML-C19-PH6 | 0 | 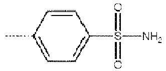 | -- | H | H | H | NO₂ |

It is also disclosed a method for inhibiting PD-L1 expression in a subject, comprising administering an effective amount of a compound or a pharmaceutical composition of the invention to the subject. It is also disclosed a method for increasing HLA-1 expression in a subject, comprising administering an effective amount of a compound or a pharmaceutical composition of the invention to the subject. It is also disclosed a method for treatment or prevention of a PD-L1-associated cancer or sepsis or septic shock in a subject, comprising administering an effective amount of a compound or a pharmaceutical composition of the invention to a subject. Accordingly, it is also disclosed a use of a compound or a pharmaceutical composition of the invention for the manufacture of a medicament in inhibiting PD-L1 level in a subject. It is also disclosed a use of a compound or a pharmaceutical composition of the invention for the manufacture of a medicament in treatment or prevention of a PD-L1-associated cancer or sepsis or septic shock in a subject.

### Brief Description of the Drawings

Figure 1 shows the respective levels of PD-L1 and HLA-DR in a septic shock patient and a normal healthy subject.
Figure 2 shows line graphs depicting the increase in PD-L1 and decrease in HLA-DR in LPS-primed monocyte model system.
Figure 3 shows line graphs depicting the effects of ML-C19-A, ML-C19-B, ML-A1-B, or ML-A1-C on the expression levels of PD-L1 and HLA-DR in LPS-primed monocyte model system.
Figure 4 shows the dose dependency of ML-C19-A, ML-C19-B, ML-A1-B, or ML-A1-C on the level of PD-L1 in LPS-primed monocyte model system.
Figure 5 shows the Effect on PD-L1 expression on IFN-γ stimulated B16F10 cancer line. IFN-γ stimulated-monocytes were incubated in the presence of either a DMSO vehicle control, ML-A1-B or ML-A1-C for 1 day. Treated cells were harvested and the PD-L1 molecules were analyzed using flow cytometry. Surface molecule expression is presented as the MFI relative to each day that the cells were treated with the DMSO vehicle control medium. Values are presented as the mean ± SEM from 3 independent experiments.
Figure 6 shows the direct cytotoxicity to B16F10 cancer cell line and human PBMCs. The B16F10 and human PBMCs were incubated in the presence of various concentrations of ML-A1-B and ML-A1-C compounds for 1 day. The cell cytotoxicity was monitored through MTS assay. Optical density of DMSO control groups was taken as 100 % of cell viability. Values are presented as the mean ± SEM from 3 independent experiments.
Figure 7 shows the lung metastases after i.v. injection of B16F10 tumor cells. C57BL/6 (n = 11/group) mice were injected i.v. with 2 × 10⁵ of the B16F10 tumor cells. Two mg/kg of ML-A1-B, ML-A1-C, or with DMSO vehicle control were used. The animals were killed on day 14 and tumor nodules in the lungs were counted. Data are presented as the number of each individual mouse and the mean of each group. Representative photographs of the lung of each group are also shown (left).
Figure 8 shows the lung metastases after i.v. injection of B16F10 tumor cells. NOD/SCID (n = 5/group) mice were injected i.v. with 2 × 10⁵ of the B16F10 tumor cells. Two mg/kg of ML-A1-B, ML-A1-C, or with DMSO vehicle control were used. The animals were killed on day 14 and tumor nodules in the lungs were counted. Data are presented as the number of each individual mouse and the mean of each group. Representative photographs of the lung of each group are also shown (left).
Figure 9 shows toxic assay results of the compounds ML-A1-B and ML-A1-C.
Figure 10 shows the combination therapy of anti-PD-1 antibody with compounds, ML-C19-A and ML-A1-C.

### Detailed Description of the Invention

The invention found a series of benzo-heterocyclic derivatives and their applications in inhibiting PD-L1 expression and increasing HLA-DR expression. Accordingly, the compounds are able to treat and/or prevent PD-L1 associated diseases such as PD-L1-associated cancers and sepsis or septic shock. Particularly, the cancer treatment through the compounds involves cancer immunotherapy rather than cytotoxicity. In other words, the compounds are able to immunologically treat cancers without killing the cells.

The detailed description provided below in connection with the appended drawings is intended as a description of the present examples and is not intended to represent the only forms in which the present examples may be constructed or utilized. The description sets forth the functions of the examples and the sequence of steps for constructing and operating the examples. However, the same or equivalent functions and sequences may be accomplished by different examples.

### Definitions

For convenience, certain terms employed in the context of the present disclosure are collected here. Unless defined otherwise, all technical and scientific terms used herein have the same meaning as commonly understood by one of the ordinary skill in the art to which this invention belongs.

The singular forms "a," "an," and "the" are used herein to include plural referents unless the context clearly dictates otherwise.

The term "treatment" or "treating" as used herein is intended to mean obtaining a desired pharmacological and/or physiologic effect, e.g., ameliorating the symptoms associated with a disease. The effect may be prophylactic in terms of completely or partially preventing a disease or symptom thereof and/or therapeutic in terms of a partial or complete cure for a disease and/or adverse effect attributable to the disease. "Treatment" as used herein includes preventative (e.g., prophylactic), curative or palliative treatment of a disease in a mammal, particularly human; and includes: (1) preventative (e.g., prophylactic), curative or palliative treatment of a disease or condition (e.g., a cancer or heart failure) from occurring in an individual who may be pre-disposed to the disease but has not yet been diagnosed as having it; (2) inhibiting a disease (e.g., by arresting its development); or (3) relieving a disease (e.g., reducing symptoms associated with the disease).

The term "administered," "administering" or "administration" are used interchangeably herein to refer a mode of delivery, including, without limitation, intravenously, intramuscularly, intraperitoneally, intraarterially, subcutaneously, or transdermally administering an agent (e.g., compound or a composition) of the present invention.

As used herein, the terms "prevent," "preventing" and "prevention" refer to the prevention of the onset, recurrence or spread of a disease or disorder, or of one or more symptoms thereof. In certain embodiments, the terms refer to the treatment with or administration of a compound or an antibody or dosage form provided herein, with or without one or more other additional active agent(s), prior to the onset of symptoms, particularly to patients at risk of disease or disorders provided herein. The terms encompass the inhibition or reduction of a symptom of the particular disease. In this regard, the term "prevention" may be interchangeably used with the term "prophylactic treatment.

The term "an effective amount" as used herein refers to an amount effective at a certain dosage and period of time to achieve the desired result with respect to the treatment of a disease. For example, in the treatment of sepsis, an agent (i.e., a compound or a composition) which decreases, prevents, delays, suppresses or arrests any symptoms of sepsis would be effective. An effective amount of an agent is not required to cure a disease or condition but will provide a treatment for a disease or condition such that the onset of the disease or condition is delayed, hindered or prevented, or the disease or condition symptoms are ameliorated. The effective amount may be a single dose or divided into two or more doses in a suitable form to be administered at one, two or more times throughout a designated time period.

The term "subject" or "patient" refers to an animal, including the human species, that is treatable with the method of the present invention. The term "subject" or "patient" is intended to refer to both the male and female gender unless one gender is specifically indicated. Accordingly, the term "subject" or "patient" comprises any mammal which may benefit from the treatment method of the present disclosure.

As used herein, the term "anticancer agent" or "cancer therapeutic agent" is meant to include anti-proliferative agents and chemotherapeutic agents.

As used herein, the terms "co-administration" and "in combination with" include the administration of two or more therapeutic agents simultaneously, concurrently or sequentially within no specific time limits unless otherwise indicated. In one embodiment, the therapeutic agents are in the same composition or unit dosage form. In other embodiments, the therapeutic agents are in separate compositions or unit dosage forms.

The term "pharmaceutically acceptable" is employed herein to refer to those compounds, materials, compositions, and/or dosage forms which are, within the scope of medical judgment, suitable for use in contact with the tissues of human beings and animals without excessive toxicity, irritation, allergic response, or other problem or complication, and commensurate with a reasonable benefit/risk ratio.

As used herein, "pharmaceutically acceptable salts" refers to derivatives of the disclosed compounds wherein the parent compound is modified by making acid or base salts thereof. Examples of pharmaceutically acceptable salts include, but are not limited to, mineral or organic acid salts of basic residues such as amines, pyridine, pyrimidine and quinazoline; alkali or organic salts of acidic residues such as carboxylic acids; and the like.

As used herein, the term "pharmaceutically acceptable carrier" refers to any of the standard pharmaceutical carriers, e.g., a phosphate buffered saline solution, water, and emulsions, such as an oil/water or water/oil emulsion, and various types of wetting agents. The compositions may also include stabilizers and preservatives and any of the above noted carriers with the additional proviso that they be acceptable for use in vivo. For examples of carriers, stabilizers and adjuvants, see Martin REMINGTON'S PHARM. SCI., 18th Ed., Mack Publ. Co., Easton, Pa. (1995), and the "PHYSICIAN'S DESK REFERENCE," 58th ed., Medical Economics, Montvale, N.J. (2004).

As used herein, the term "stereoisomer" is a general term for all isomers of individual molecules that differ only in the orientation of their atoms in space. It includes enantiomers and isomers of compounds with more than one chiral center that are not mirror images of one another (diastereoisomers).

The term "chiral center" refers to a carbon atom to which four different groups are attached.

The terms "enantiomer" and "enantiomeric" refer to a molecule that cannot be superimposed on its mirror image and hence is optically active, wherein the enantiomer rotates the plane of polarized light in one direction and its mirror image compound rotates the plane of polarized light in the opposite direction.

As used herein, halo or halogen refers to fluoro, chloro, bromo or iodo. When the term "halo" is used as a prefix of, for example, a hydrocarbyl, it means that the hydrocarbyl is substituted by at least one halogen.

As used herein, the term "hydrocarbyl" refers to a univalent group formed by removing a hydrogen atom from a hydrocarbon, such as alkyl, alkenyl, alkynyl, etc. as further illustrated as follows.

As used herein, the term "alkyl" refers to straight or branched hydrocarbon chains containing the specified number of carbon atoms. For example, "C₁-C₆ alkyl" is selected from straight-chained and branched non-cyclic hydrocarbons having from 1 to 6 carbon atoms. Representative straight chain C₁-C₆ alkyl groups include -methyl, -ethyl, -n-propyl, -n-butyl, -n-pentyl, and -n-hexyl. Representative branched C₁-C₆ alkyls include -isopropyl, -sec-butyl, - isobutyl, -tert-butyl, -isopentyl, -neopentyl, 1-methylbutyl, 2-methylbutyl, 3-methylbutyl, 1,1-dimethylpropyl, 1,2-dimethylpropyl, 1-methylpentyl, 2-methylpentyl, 3-methylpentyl, 4-methylpentyl, 1-ethylbutyl, 2-ethylbutyl, 3-ethylbutyl, 1,1-dimethylbutyl, 1,2-dimethylbutyl, 1,3-dimethylbutyl, 2,2-dimethylbutyl, 2,3-dimethylbutyl, and 3,3-dimethylbutyl.

As used herein, the term "alkenyl" refers to straight or branched chain hydrocarbon chains containing the specified number of carbon atoms and one or more double bonds. For example, "C₂-C₆ alkenyl" is selected from straight chain and branched non-cyclic hydrocarbons having from 2 to 6 carbon atoms and including at least one carbon-carbon double bond. Representative straight chain and branched C₂-C₆ alkenyl groups include -vinyl, -allyl, -1-butenyl, -2-butenyl, -isobutylenyl, -1-pentenyl, -2-pentenyl, -3-methyl-1-butenyl, -2-methyl-2-butenyl, -2,3-dimethyl-2-butenyl, -1-hexenyl, 2-hexenyl, and 3-hexenyl.

As used herein, the term "alkynyl" refers to straight or branched chain hydrocarbon chains containing the specified number of carbon atoms and one or more triple bonds. For example, "C₂-C₆ alkynyl" is selected from straight chain and branched non-cyclic hydrocarbon having from 2 to 6 carbon atoms and including at least one carbon-carbon triple bond. Representative straight chain and branched C₂-C₆ alkynyl groups include -acetylenyl, -propynyl, -1-butyryl, -2-butyryl, -1-pentynyl, -2-pentynyl, -3-methyl-1-butynyl, -4-pentynyl, -1-hexynyl, - 2-hexynyl, and -5-hexynyl.

As used herein, "cycloalkyl" refers to a group selected from C₃-C₁₂ cycloalkyl, and preferably a C₃₋₈ cycloalkyl. Typical cycloalkyl groups include cyclopropyl, cyclobutyl, cyclopentyl, cyclohexyl, cycloheptyl, cyclooctyl, and cyclononyl.

As used herein, the term "alkoxy" refers to a straight or branched alkoxy group containing the specified number of carbon atoms. For example, C₁₋₆alkoxy means a straight or branched alkoxy group containing at least 1, and at most 6, carbon atoms. Examples of "alkoxy" as used herein include, but are not limited to, methoxy, ethoxy, propoxy, prop-2-oxy, butoxy, but-2-oxy, 2-methylprop-1-oxy, 2-methylprop-2-oxy, pentoxy and hexyloxy.

As used herein, the term "alkylthio" (also termed as alkylsulfanyl) refers to straight-chain or branched alkyl groups (preferably having 1 to 6 carbon atoms, e.g. 1 to 4 carbon atoms (C₁-C₆-alkylthio), which are bound to the remainder of the molecule via a sulfur atom at any bond in the alkyl group. Examples of C₁-C₄-alkylthio include methylthio, ethylthio, n-propylthio, isopropylthio, *n*-butylthio, sec-butylthio, isobutylthio and *tert*-butylthio. Examples of C₁-C₆-alkylthio include, apart from those mentioned for C₁-C₄-alkylthio, 1-, 2- and 3-pentylthio, 1-, 2- and 3-hexylthio and the positional isomers thereof.

As used herein, the term "alkoxyalkyl" refers to the group -alk₁-O-alk₂ where alk₁ is alkyl or alkenyl, and alk₂ is alkyl or alkenyl.

As used herein, the term "(di)alkylamino" refers to the group --NRR' where R is alkyl and R' is hydrogen or alkyl.

As used herein, "aryl" refers to a group selected from C₆₋₁₄ aryl, especially C₆₋₁₀ aryl. Typical C₆₋₁₄ aryl groups include phenyl, naphthyl, phenanthryl, anthracyl, indenyl, azulenyl, biphenyl, biphenylenyl and fluorenyl groups.

As used herein, "heteroaryl" refers to a group having from 5 to 14 ring atoms; 6, 10 or 14 pi electrons shared in a cyclic array; and containing carbon atoms and 1, 2 or 3 oxygen, nitrogen and/or sulfur heteroatoms. Examples of heteroaryl groups include indazolyl, furyl, thienyl, pyrrolyl, imidazolyl, oxazolyl, thiazolyl, imidazolyl, pyrazolyl, isoxazolyl, isothiazolyl, oxadiazolyl, triazolyl, thiadiazolyl, pyridyl, pyridazinyl, pyrimidinyl, pyrazinyl, tetrazolyl, triazinyl, azepinyl, oxazepinyl, morpholinyl, thiazepinyl, diazepinyl, thiazolinyl, benzimidazolyl, benzoxazolyl, imidazopyridinyl, benzoxazinyl, benzothiazinyl, benzothiophenyl oxazolopyridinyl, benzofuranyl, quinolinyl, quinazolinyl, quinoxalinyl, benzothiazolyl, phthalimido, benzofuranyl, benzodiazepinyl, indolyl, indanyl, azaindazolyl, deazapurinyl and isoindolyl.

As used herein, the term "amino" or "amino group" refers to --NH₂. When the term "amino" is used as a prefix of, for example, a hydrocarbyl, it means that the hydrocarbyl is substituted by at least one amino group.

As used herein, the term "cyano" refers to -C=N. When the term "cyano" is used as a prefix of, for example, a hydrocarbyl, it means that the hydrocarbyl is substituted by at least one cyano group.

As used herein, the term "nitro" refers to -NO₂. When the term "nitro" is used as a prefix of, for example, a hydrocarbyl, it means that the hydrocarbyl is substituted by at least one nitro group.

As used herein, the term "hydroxy" refers to -OH. When the term "hydroxy" is used as a prefix of, for example, a hydrocarbyl, it means that the hydrocarbyl is substituted by at least one hydroyl group.

As used herein, the term "optionally substituted" refers to a group that is unsubstituted or substituted with one or more substituents. For example, where the groups C₁-C₆ alkyl, C₂-C₆ alkenyl, C₂-C₆ alkynyl, --O--C₁-C₆ alkyl, --O--C₂-C₆ alkenyl, and --O--C₂-C₅ alkynyl are referred to as being optionally substituted, they may or may not be substituted.

### Compounds of the Invention

The invention concerns a compound having the following Formula (I), 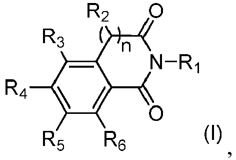 wherein the compound is selected from the group consisted of the following:

| Compound Name | n | R₁ | R₂ | R₃ | R₄ | R₅ | R₆ |
|---|---|---|---|---|---|---|---|
| ML-A1-B | 1 | -CH₂CH₂N(CH₃)₂ | =CH-CH=CH- | | 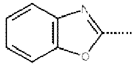 | H | H |

or a tautomer, stereoisomer or enantiomer thereof, or a solvate, or a pharmaceutically acceptable salt thereof.

According to an embodiment of the invention, the compound is 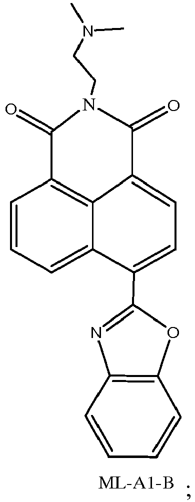 or a tautomer, a stereoisomer, an enantiomer, or a solvate thereof, or a pharmaceutically acceptable salt thereof.

In one aspect, it is disclosed a compound having the following Formula (I), 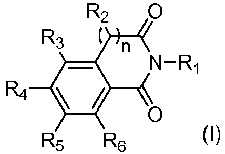 wherein
n is 0 or 1,
R₁ is selected from halogen, C₁₋₁₀alkyl, C₂₋₁₀alkenyl, C₂₋₁₀alkynyl, haloC₁₋₁₀alkyl, hydroxyC₁₋₁₀alkyl, aminoC₁₋₁₀alkyl, C₃₋₁₀cycloalkyl, 3- to 10-membered cyclic heterocycloalkyl having 1 to 3 heteroatoms selected from N, S and O, C₆₋₁₀aryl or 5- to 10-membered mono- or bi-cyclic heteroaryl having from 1 to 3 heteroatoms selected from N, S and O, wherein the amino moiety of aminoalkyl is unsubstituted or substituted by one or two alkyl groups, and the cycloalkyl, heterocycloalkyl, aryl and heteroaryl are substituted by -SO₂NH₂, or -CONH₂;
R₂ is selected from hydrogen, halogen, hydroxy, amino, cyano, nitro, C₁₋₁₀alkyl, C₂₋₁₀alkenyl, C₂₋₁₀alkynyl, C₁₋₁₀alkoxy, C₁₋₁₀alkylthio, C₁₋₁₀alkylamino, haloC₁₋₁₀alkyl, hydroxyC₁₋₁₀alkyl, aminoC₁₋₁₀alkyl, C₃₋₁₀cycloalkyl, 3 to 10 membered cyclic heterocycloalkyl having 1 to 3 heteroatoms selected from N, S and O, C₆₋₁₀aryl or 5 to 10 membered mono- or bi-cyclic heteroaryl having from 1 to 3 heteroatoms selected from N, S and O;
R₃ is selected from hydrogen, halogen, hydroxy, amino, cyano, nitro, C₁₋₁₀alkyl, C₂₋₁₀alkenyl, C₂₋₁₀alkynyl, C₁₋₁₀alkoxy, C₁₋₁₀alkylthio, C₁₋₁₀alkylamino, haloC₁₋₁₀alkyl, hydroxyC₁₋₁₀alkyl, aminoC₁₋₁₀alkyl, C₃₋₁₀cycloalkyl, 3 to 10 membered cyclic heterocycloalkyl having 1 to 3 heteroatoms selected from N, S and O, C₆₋₁₀aryl or 5 to 10 membered mono- or bi-cyclic heteroaryl having from 1 to 3 heteroatoms selected from N, S and O; or
R₂ and R₃ together with carbon atoms to which they attach form a benzene ring, wherein the benzene ring is unsubstituted or substituted by one or more groups independently selected from halogen, hydroxy, amino, cyano, nitro, C₁₋₁₀alkyl, C₂₋₁₀alkenyl, C₂₋₁₀alkynyl, C₁₋₁₀alkoxy, C₁₋₁₀alkylthio, C₁₋₁₀alkylamino, haloC₁₋₁₀alkyl, hydroxyC₁₋₁₀alkyl, aminoC₁₋₁₀alkyl, C₃₋₁₀cycloalkyl, 3 to 10 membered cyclic heterocycloalkyl having 1 to 3 heteroatoms selected from N, S and O, C₆₋₁₀aryl or 5 to 10 membered mono- or bi-cyclic heteroaryl having from 1 to 3 heteroatoms selected from N, S and O, with the proviso that n is 1; and
R₄, R₅ and R₆ are each independently selected from hydrogen, halogen, hydroxy, amino, cyano, nitro, C₁₋₁₀alkyl, C₂₋₁₀alkenyl, C₂₋₁₀alkynyl, C₁₋₁₀alkoxy, C₁₋₁₀alkylthio, C₁₋₁₀alkylamino, haloC₁₋₁₀alkyl, hydroxyC₁₋₁₀alkyl, aminoC₁₋₁₀alkyl, C₃₋₁₀cycloalkyl, 3 to 10 membered cyclic heterocycloalkyl having 1 to 3 heteroatoms selected from N, S and O, C₆₋₁₀aryl or 5- to 10-membered mono- or bi-cyclic heteroaryl having from 1 to 3 heteroatoms selected from N, S and O; wherein the cycloalkyl, heterocycloalkyl, aryl and heteroaryl in R₂ to R₆ may be unsubstituted or substituted by one to four groups selected from halogen, hydroxy, amino, cyano, nitro, C₁₋₁₀alkyl, C₂₋₁₀alkenyl, C₂₋₁₀alkynyl, C₁₋₁₀alkoxy, C₁₋₁₀alkylthio, C₁₋₁₀alkylamino, haloC₁₋₁₀alkyl, hydroxyC₁₋₁₀alkyl, aminoC₁₋₁₀alkyl or C₃₋₁₀cycloalkyl;
with provisos that (1) when n is 1, R₁ is 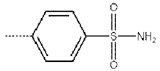 and R₂ and R₃ together with carbon atoms to which they attach form a benzene ring, R₄, R₅ and R₆ cannot simltaneously represent hydrogen; (2) when n is 0 and R₁ is selected from the cycloalkyl, heterocycloalkyl, aryl or heteroaryl, R₃, R₄, R₅ and R₆ are not simultaneously hydrogen; (3) when n is 0, R₁ is 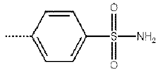 and R₃ and R₆ simultaneously represent hydrogen, if one of R₄ and R₅ is hydrogen, the other cannot be methyl, tert-butyl or nitro; (4) when n is 0, R₁ is 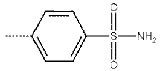 and R₃ and R₆ simultaneously represent hydrogen, R₄ and R₅ cannot simultaneously represent chloro; and (5) when n is 0 and R₁ is 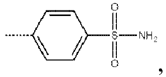 R₃, R₄, R₅ and R₆ cannot simultaneously represent chloro;
or a tautomer, stereoisomer or enantiomer thereof, or a solvate, prodrug or a pharmaceutically acceptable salt thereof.

In some embodiments of disclosed Formula (I), n is 1; R₁ is (C₁₋₁₀(di)alkylamino)C₁₋₁₀alkyl, hydroxyC₁₋₁₀alkyl or C₆₋₁₀aryl substituted by -SO₂NH₂ or -CO₂NH₂; R₂ and R₃ together with carbon atoms to which they attach form a benzene ring, wherein the benzene ring is unsubstituted or substituted by one or more groups independently selected from halogen, amino, cyano, nitro or C₁₋₁₀alkyl; R₄ is H, halogen or 5 to 10 membered mono- or bi-cyclic, unsubstituted or substituted heteroaryl having from 1 to 3 heteroatoms selected from N, S and O; and R₅ and R₆ are each independently hydrogen, halogen, amino, cyano, nitro, C₁₋₁₀alkyl or haloC₁₋₁₀alkyl. Preferably, n is 1, R₁ is (C₁₋₆(di)alkylamino)C₁₋₆alkyl, hydroxyC₁₋₆alkyl or phenyl substituted by -SO₂NH₂; R₂ and R₃ together with carbon atoms to which they attach form an unsubstituted benzene ring; R₄ is H, halogen or 9 to 10 membered bi-cyclic, unsubstituted or substituted heteroaryl having from two heteroatoms selected from N, S and O; and R₅ and R₆ are each independently hydrogen, halogen, nitro or C₁₋₆alkyl. More preferably, n is 1, R₁ is - CH₂CH₂N(CH₃)₂, -CH₂CH₂OH, -(CH₂)₃CH₃ or phenyl substituted by -SO₂NH₂; R₂ and R₃ together with carbon atoms to which they attach form an unsubstituted benzene ring; R₄ is 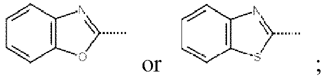 and R₅ and R₆ are each independently hydrogen, halogen, nitro or C₁₋₄alkyl.

In some embodiments of disclosed Formula (I), n is 0, R₁ is C₆₋₁₀aryl substituted by - SO₂NH₂ or -CO₂NH₂; R₃, R₄, R₅ and R₆ are each independently selected from hydrogen, halogen, amino, cyano, nitro or C₁₋₁₀alkyl. Preferably, n is 0, R₁ is phenyl substituted by - SO₂NH₂; R₃ and R₆ are each independently hydrogen, halogen or C₁₋₆alkyl; and R₄ and R₅ are each independently selected from hydrogen, halogen, nitro or C₁₋₆alkyl. More preferably, n is 0, R₁ is phenyl substituted by -SO₂NH₂; R₃ and R₆ are each independently selected from hydrogen, halogen or C₁₋₄alkyl; and R₄ and R₅ are each independently selected from hydrogen, halogen, nitro or C₁₋₄alkyl. More preferably, n is 0, R₁ is phenyl substituted by -SO₂NH₂; R₃ and R₆ are each independently selected from hydrogen or halogen; and R₄ and R₅ are each independently selected from hydrogen, halogen, nitro or C₁₋₄alkyl.

According to a disclosure, the compounds include but are not limited to the following: 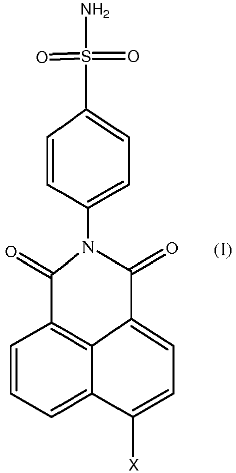 wherein X is Br, i.e., 4-(6-bromo-1,3-dioxo-1H-benzo[de]isoquinolin-2(3H)-yl)benzenesulfonamide (compound ML-C19-B); or a tautomer, stereoisomer or enantiomer thereof, or a solvate, prodrug or a pharmaceutically acceptable salt thereof.

According to a disclosure, the compounds include but are not limited to the following: 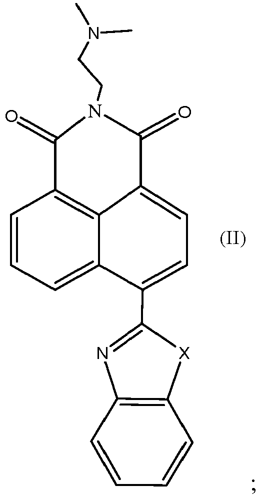 wherein X is O or S or N; or a tautomer, stereoisomer or enantiomer thereof, or a solvate, prodrug or a pharmaceutically acceptable salt thereof.

According to a disclosure, the compounds include but are not limited to the following:

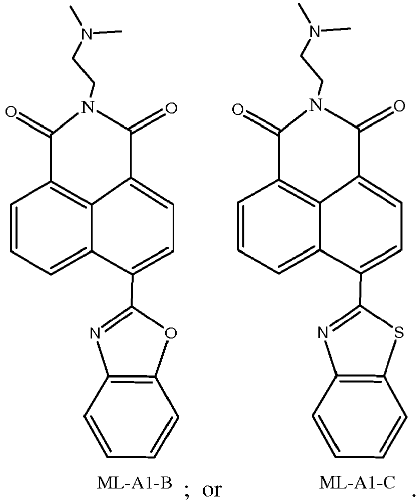

Compound ML-C19-B is prepared by heating with 1,8-naphthanhydride and sulfanilamide under reflux condition and may be prepared by the method set forth in working example 1.1 of the present disclosure.

Both compounds ML-A1-B and ML-A1-C derive from 6-bromo-2-(2-(dimethylamino)ethyl)-1H-benzo[de]isoquinoline-1,3-(2H)-dione (compound A1), and may be prepared by the method set forth in working example 1.2 of the present disclosure.

Further, according to the present invention, all compounds of the present invention are found to possess the ability to decrease PD-L1 level and optionally increase HLA-DR level.

The invention disclosed herein also encompasses prodrugs of the disclosed compounds. Prodrugs are considered to be any covalently bonded carriers that release an active compound of Formula (I) *in vivo.* Non-limiting examples of prodrugs include esters of compounds of Formula (I), and these may be prepared by reacting such compounds with anhydrides such as succinic anhydride.

The invention disclosed herein also encompasses pharmaceutically acceptable salts of the disclosed compounds. In one embodiment, the present invention includes any and all nontoxic, pharmaceutically acceptable salts of the disclosed compounds, comprising inorganic and organic acid addition salts and basic salts. The pharmaceutically acceptable salts of the present invention can be synthesized from the parent compound which contains a basic or acidic moiety by conventional chemical methods. Generally, such salts can be prepared by reacting the free acid or base forms of these compounds with a sufficient amount of the appropriate base or acid in water or in an organic diluent like ether, ethyl acetate, ethanol, isopropanol, or acetonitrile, or a mixture thereof. For example, such salts include acetates, ascorbates, benzenesulfonates, benzoates, besylates, bicarbonates, bitartrates, bromides/hydrobromides, Ca-edetates/edetates, camsylates, carbonates, chlorides/hydrochlorides, citrates, edisylates, ethane disulfonates, estolates esylates, fumarates, gluceptates, gluconates, glutamates, glycolates, glycollylarsnilates, hexylresorcinates, hydrabamines, hydroxymaleates, hydroxynaphthoates, iodides, isothionates, lactates, lactobionates, malates, maleates, mandelates, methanesulfonates, mesylates, methylbromides, methylnitrates, methylsulfates, mucates, napsylates, nitrates, oxalates, pamoates, pantothenates, phenylacetates, phosphates/diphosphates, polygalacturonates, propionates, salicylates, stearates subacetates, succinates, sulfamides, sulfates, tannates, tartrates, teoclates, toluenesulfonates, triethiodides, ammonium, benzathines, chloroprocaines, cholines, diethanolamines, ethylenediamines, meglumines and procaines. Further pharmaceutically acceptable salts can be formed with cations from metals like aluminium, calcium, lithium, magnesium, potassium, sodium, zinc and the like. (See Pharmaceutical salts, Birge, S. M. et al., J. Pharm. Sci., (1977), 66, 1-19.)

The invention disclosed herein also encompasses solvates of the disclosed compounds. One type of solvate is a hydrate. Solvates typically do not contribute significantly to the physiological activity or toxicity of the compounds and as such can function as pharmacological equivalents.

The invention disclosed herein also encompasses tautomers and isomers of the disclosed compounds. A given chemical formula or name shall encompass tautomers and all stereo, optical and geometrical isomers (e.g. enantiomers, diastereomers, E/Z isomers etc.) and racemates thereof as well as mixtures in different proportions of the separate enantiomers, mixtures of diastereomers, or mixtures of any of the foregoing forms where such isomers and enantiomers exist, as well as salts, including pharmaceutically acceptable salts thereof and solvates thereof such as, for instance, hydrates including solvates of the free compounds or solvates of a salt of the compound.

### Preparation of the Compounds of the Invention

The compounds of the present invention can be prepared using methods known to those skilled in the art in view of this disclosure. For example, the general scheme in synthesis of the compounds of the invention is shown as follows:

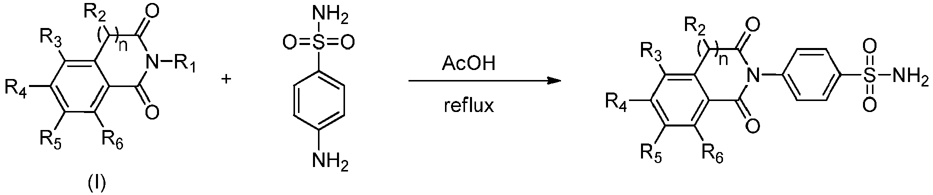

The general scheme in syntheses of the preferred compounds of the invention, ML-A1-B, ML-A1-C, and other derivatives is shown as follows:

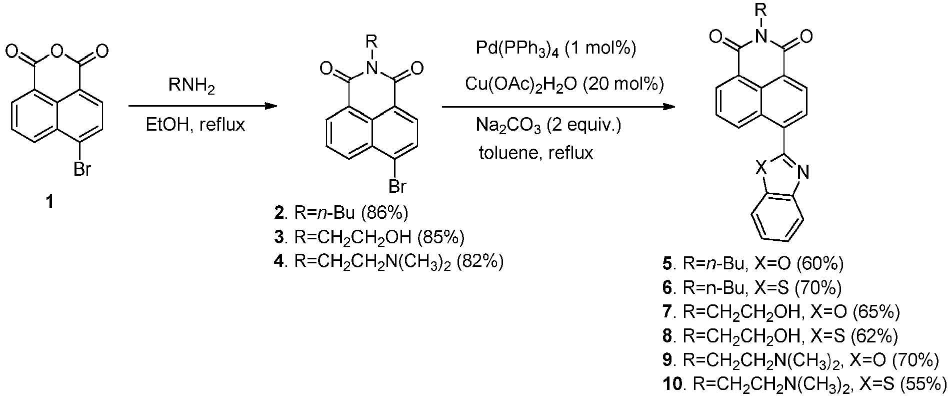

The general scheme in syntheses of the preferred compounds of the invention, ML-C19-PH2, ML-C19-PH3, ML-C19-PH4 and ML-C19-PH6 is shown as follows: 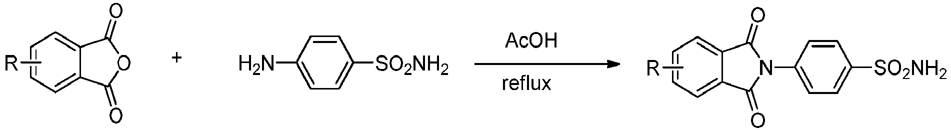 R=H, Br, CH₃ or NO₂

### Pharmaceutical Compositions

The compounds of the present invention are found to possess the ability to reverse the unique characteristics of a septic or cancer patient, i.e., an increase in PD-L1 level and optionally a decrease in HLA-DR level, suggesting that the compounds of the invention can be used in cancer immunotherapy and treatment or prevention of sepsis or septic shock and cancers.

In one aspect, the invention provides a pharmaceutical composition comprising an effective amount of a compound of the invention and a pharmaceutically acceptable carrier. In some embodiments, the amount of the compound ranges from 50 µg to 500 µg, preferably, 100 µg to 300 µg or 100 µg to 200µg.

Preferably, the compound or composition of the invention may be formulated into liquid pharmaceutical compositions, which are sterile solutions, or suspensions that can be administered by, for example, intravenous, intramuscular, subcutaneous, or intraperitoneal injection. Suitable diluents or solvent for manufacturing sterile injectable solution or suspension include, but are not limited to, 1,3-butanediol, mannitol, water, Ringer's solution, and isotonic sodium chloride solution. Fatty acids, such as oleic acid and its glyceride derivatives, are also useful for preparing injectables, as are natural pharmaceutically-acceptable oils, such as olive oil or castor oil. These oil solutions or suspensions may also contain alcohol diluent or carboxymethyl cellulose or similar dispersing agents. Other commonly used surfactants such as Tweens or Spans or other similar emulsifying agents or bioavailability enhancers that are commonly used in manufacturing pharmaceutically acceptable dosage forms can also be used for the purpose of formulation. Oral administration may be either liquid or solid composition form.

In some embodiments, the pharmaceutical composition of the invention further comprises a second active agent. In some preferred embodiments, the second active agent is an anticancer agent or an immune checkpoint inhibitor. In some embodiments, the immune checkpoint inhibitor is a PD-L1 inhibitor, PD-1 inhibitor or CTLA-1 inhibitor.

In some embodiments, the second anticancer agent includes, but is not limited to, an antimetabolite (e.g., 5-fluoro uracil, methotrexate, fludarabine, cytarabine (also known as cytosine arabinoside or Ara-C), and high dose cytarabine), antimicrotubule agent (e.g., vinca alkaloids, such as vincristine and vinblastine; and taxane, such as paclitaxel and docetaxel), alkylating agent (e.g., mechlorethamine, chlorambucil, cyclophosphamide, melphalan, melphalan, ifosfamide, carmustine, azacitidine, decitabine, busulfan, cyclophosphamide, dacarbazine, ifosfamide, and nitrosoureas, such as carmustine, lomustine, bischloroethylnitrosurea, and hydroxyurea), platinum agent (e.g., cisplatin, carboplatin, oxaliplatin, satraplatin (JM-216), and CI-973), anthracycline (e.g., doxorubicin and daunorubicin), antitumor antibiotic (e.g., mitomycin, bleomycin, idarubicin, adriamycin, daunomycin (also known as daunorubicin, rubidomycin, or cerubidine), and mitoxantrone), topoisomerase inhibitor (e.g., etoposide and camptothecin), purine antagonist or pyrimidine antagonist (e.g., 6-mercaptopurine, 5-fluorouracil, cytarabine, clofarabine, and gemcitabine), cell maturing agent (e.g., arsenic trioxide and tretinoin), DNA repair enzyme inhibitor (e.g., podophyllotoxine, etoposide, irinotecan, topotecan, and teniposide), enzyme that prevents cell survival (e.g., asparaginase and pegaspargase), histone deacetylase inhibitors (e.g., vorinostat), any other cytotoxic agents (e.g., estramustine phosphate, dexamethasone, prednimustine, and procarbazine), hormone (e.g., dexamethasone, prednisone, methylprednisolone, tamoxifen, leuprolide, flutamide, and megestrol), monoclonal antibody (e.g., gemtuzumab ozogamicin, alemtuzumab, rituximab, and yttrium-90-ibritumomab tiuxetan), immuno-modulator (e.g., thalidomide and lenalidomide), Bcr-Abl kinase inhibitor (e.g., AP23464, AZD0530, CGP76030, PD180970, SKI-606, imatinib, BMS354825 (dasatinib), AMN107 (nilotinib), and VX-680), hormone agonist or antagonist, partial agonist or partial antagonist, kinase inhibitor, surgery, radiotherapy (e.g., gamma-radiation, neutron bean radiotherapy, electron beam radiotherapy, proton therapy, brachytherapy, and systemic radioactive isotopes), endocrine therapy, biological response modifiers (e.g., interferons, interleukins, and tumor necrosis factor), hyperthermia and cryotherapy, and agents to attenuate any adverse effect (e.g., antiemetics). In one embodiment, the anticancer agent or cancer therapeutic agent is a cytotoxic agent, an anti-metabolite, an antifolate, an HDAC inhibitor such as MGCD0103 (a.k.a. N-(2-aminophenyl)-4-((4-(pyridin-3-yl)pyrimidin-2-ylamino)methyl)benzamid- e), a DNA intercalating agent, a DNA cross-linking agent, a DNA alkylating agent, a DNA cleaving agent, a topoisomerase inhibitor, a CDK inhibitor, a JAK inhibitor, an anti-angiogenic agent, a Bcr-Abl inhibitor, an HER2 inhibitor, an EGFR inhibitor, a VEGFR inhibitor, a PDGFR inhibitor, an HGFR inhibitor, an IGFR inhibitor, a c-Kit inhibitor, a Ras pathway inhibitor, a PI3K inhibitor, a multi-targeted kinase inhibitor, an mTOR inhibitor, an anti-estrogen, an anti-androgen, an aromatase inhibitor, a somatostatin analog, an ER modulator, an anti-tubulin agent, a vinca alkaloid, a taxane, an HSP inhibitor, a Smoothened antagonist, a telomerase inhibitor, an anti-metastatic agent, an immunosuppressant, a biologic such as antibody or hormonal therapy.

In some embodiments of the invention, the PD-L1 inhibitor is a small interfering RNA (siRNA) of PD-L1, a small hairpin (sh)RNA of PD-L1 or an antisense RNA of PD-L1, an anti-PD-Ll antibody such as lambrolizumab, MPDL3280A, MEDI4736 and avelumab or an antigen-binding fragment thereof, that binds to a PD-L1 protein.

In some embodiments, the PD-1 inhibitor includes a small interfering RNA (siRNA) of PD-1, a small hairpin (sh)RNA of PD-1 or an antisense RNA of PD-1, an anti-PD-1 antibody such as lambrolizumab, nivolumab, or an antigen-binding fragment thereof, that binds to a PD-1 protein.

In some embodiments, the CTLA-4 inhibitor is a small interfering RNA (siRNA) of CTLA-4, a small hairpin (sh)RNA of CTLA-4 or an antisense RNA of CTLA-4,an anti-CTLA-4 antibody such as ipilimumab. or an antigen-binding fragment thereof, that binds to a CTLA-4 protein.

### Treatment Methods

In another aspect, it is disclosed a method for inhibiting PD-L1 level in a subject, comprising administering an effective amount of a compound of the following formula or a pharmaceutical composition of the invention to the subject; 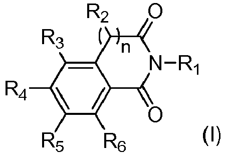 wherein
n is 0 or 1,
R₁ is selected from halogen, C₁₋₁₀alkyl, C₂₋₁₀alkenyl, C₂₋₁₀alkynyl, haloC₁₋₁₀alkyl, hydroxyC₁₋₁₀alkyl, aminoC₁₋₁₀alkyl, C₃₋₁₀cycloalkyl, 3 to 10 membered cyclic heterocycloalkyl having 1 to 3 heteroatoms selected from N, S and O, C₆₋₁₀aryl or 5 to 10 membered mono- or bi-cyclic heteroaryl having from 1 to 3 heteroatoms selected from N, S and O, wherein the amino moiety of aminoalkyl is unsubstituted or substituted by one or two alkyl groups, and the cycloalkyl, heterocycloalkyl, aryl and heteroaryl are substituted by -SO₂NH₂, or -CONH₂;
R₂ is selected from hydrogen, halogen, hydroxy, amino, cyano, nitro, C₁₋₁₀alkyl, C₂₋₁₀alkenyl, C₂₋₁₀alkynyl, C₁₋₁₀alkoxy, C₁₋₁₀alkylthio, C₁₋₁₀alkylamino, haloC₁₋₁₀alkyl, hydroxyC₁₋₁₀alkyl, aminoC₁₋₁₀alkyl, C₃₋₁₀cycloalkyl, 3 to 10 membered cyclic heterocycloalkyl having 1 to 3 heteroatoms selected from N, S and O, C₆₋₁₀aryl or 5 to 10 membered mono- or bi-cyclic heteroaryl having from 1 to 3 heteroatoms selected from N, S and O;
R₃ is selected from hydrogen, halogen, hydroxy, amino, cyano, nitro, C₁₋₁₀alkyl, C₂₋₁₀alkenyl, C₂₋₁₀alkynyl, C₁₋₁₀alkoxy, C₁₋₁₀alkylthio, C₁₋₁₀alkylamino, haloC₁₋₁₀alkyl, hydroxyC₁₋₁₀alkyl, aminoC₁₋₁₀alkyl, C₃₋₁₀cycloalkyl, 3 to 10 membered cyclic heterocycloalkyl having 1 to 3 heteroatoms selected from N, S and O, C₆₋₁₀aryl or 5 to 10 membered mono- or bi-cyclic heteroaryl having from 1 to 3 heteroatoms selected from N, S and O; or
R₂ and R₃ together with carbon atoms to which they attach form a benzene ring, wherein the benzene ring is unsubstituted or substituted by one or more groups independently selected from halogen, hydroxy, amino, cyano, nitro, C₁₋₁₀alkyl, C₂₋₁₀alkenyl, C₂₋₁₀alkynyl, C₁₋₁₀alkoxy, C₁₋₁₀alkylthio, C₁₋₁₀alkylamino, haloC₁₋₁₀alkyl, hydroxyC₁₋₁₀alkyl, aminoC₁₋₁₀alkyl, C₃₋₁₀cycloalkyl, 3 to 10 membered cyclic heterocycloalkyl having 1 to 3 heteroatoms selected from N, S and O, C₆₋₁₀aryl or 5 to 10 membered mono- or bi-cyclic heteroaryl having from 1 to 3 heteroatoms selected from N, S and O, with the proviso that n is 1; and
R₄, R₅ and R₆ are each independently selected from hydrogen, halogen, hydroxy, amino, cyano, nitro, C₁₋₁₀alkyl, C₂₋₁₀alkenyl, C₂₋₁₀alkynyl, C₁₋₁₀alkoxy, C₁₋₁₀alkylthio, C₁₋₁₀alkylamino, haloC₁₋₁₀alkyl, hydroxyC₁₋₁₀alkyl, aminoC₁₋₁₀alkyl, C₃₋₁₀cycloalkyl, 3 to 10 membered cyclic heterocycloalkyl having 1 to 3 heteroatoms selected from N, S and O, C₆₋₁₀aryl or 5 to 10 membered mono- or bi-cyclic heteroaryl having from 1 to 3 heteroatoms selected from N, S and O, wherein the cycloalkyl, heterocycloalkyl, aryl and heteroaryl in R₂ to R₆ may be unsubstituted or substituted by one to four groups selected from halogen, hydroxy, amino, cyano, nitro, C₁₋₁₀alkyl, C₂₋₁₀alkenyl, C₂₋₁₀alkynyl, C₁₋₁₀alkoxy, C₁₋₁₀alkylthio, C₁₋₁₀alkylamino, haloC₁₋₁₀alkyl, hydroxyC₁₋₁₀alkyl, aminoC₁₋₁₀alkyl, C₃₋₁₀cycloalkyl;
or a tautomer, stereoisomer or enantiomer thereof, or a solvate, prodrug or a pharmaceutically acceptable salt thereof.

According to a further object, the present invention concerns a pharmaceutical composition for use in treating or preventing a PD-L1 associated cancer in a subject by inhibiting PD-L1, wherein the pharmaceutical composition comprises the compound having the following formula (I): 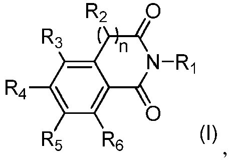 wherein the compound is selected from the group consisted of the following:

| Compound Name | n | R₁ | R₂ | R₃ | R₄ | R₅ | R₆ |
|---|---|---|---|---|---|---|---|
| ML-A1-B | 1 | -CH₂CH₂N(CH₃)₂ | =CH-CH=CH- | | 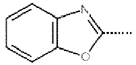 | H | H |
| ML-A1-C | 1 | -CH₂CH₂N(CH₃)₂ | =CH-CH=CH- | | 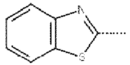 | H | H |
| ML-C19-A | 1 | 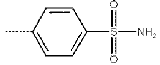 | =CH-CH=CH- | | H | H | H |

or a tautomer, stereoisomer or enantiomer thereof, or a solvate, or a pharmaceutically acceptable salt thereof.

According to a disclosure, the following compounds for use are also disclosed:

| Compound Name | n | R₁ | R₂ | R₃ | R₄ | R₅ | R₆ |
|---|---|---|---|---|---|---|---|
| ML-A1-K | 1 | -CH₂CH₂OH | =CH-CH=CH- | | 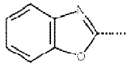 | H | H |
| ML-A1-L | 1 | -CH₂CH₂OH | =CH-CH=CH- | | 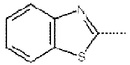 | H | H |
| ML-A1-I | 1 | -(CH₂)₃CH₃ | =CH-CH=CH- | | 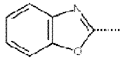 | H | H |
| ML-C19-B | 1 | 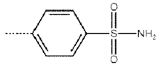 | =CH-CH=CH- | | Br | H | H |
| ML-C19-PH2 | 0 | 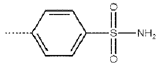 | -- | H | H | Br | H |
| ML-C19-PH3 | 0 | 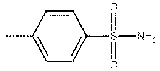 | -- | Br | Br | Br | Br |
| ML-C19-PH4 | 0 | 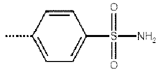 | -- | H | H | CH₃ | H |
| ML-C19-PH5 | 0 | 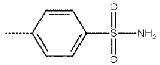 | -- | H | H | NO₂ | H |
| ML-C19-PH6 | 0 | 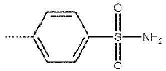 | -- | H | H | H | NO₂ |

or a tautomer, stereoisomer or enantiomer thereof, or a solvate, or a pharmaceutically acceptable salt thereof.

According to a disclosure, the compounds used in the treatment methods of the invention are as follows.

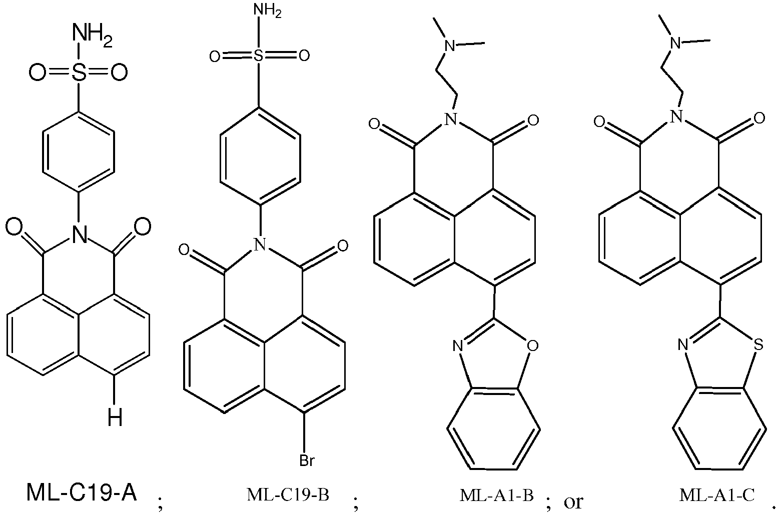

Preferably, the method can increase HLA-DR level.

In another aspect, it is disclosed a method for treatment or prevention of a PD-L1-associated cancer or sepsis or septic shock in a subject, comprising administering an effective amount of a compound as mentioned herein or a pharmaceutical composition of the invention to a subject. Preferably, the PD-L1-associated cancer is renal cell carcinoma, ovarian cancer, lung cancer, NSCLC, colon cancer, hepatocellular carcinoma or melanoma.

In some embodiments, the methods for inhibition of PD-L1 level or treatment or prevention of a PD-L1-associated cancer or sepsis or septic shock comprise an additional step of sequentially, simultaneously, separately or subsequently administering an immune checkpoint inhibitor. Preferably, the immune checkpoint inhibitor includes, but is not limited to, an anti-PD-L1 antibody, an anti-PD-1 antibody and an anti-CTLA-4 antibody. Preferably, the immune checkpoint inhibitor is ipilimumab, lambrolizumab, MPDL3280A, MEDI4736 or avelumab.

The compound or composition of the invention may be administered to a mammal, preferably human, by any route that may effectively transport the compound or composition of the invention to the appropriate or desired site of action, such as oral, nasal, pulmonary, transdermal, such as passive or iontophoretic delivery, or parenteral, e.g., rectal, depot, subcutaneous, intravenous, intramuscular, intranasal, intra-cerebella, ophthalmic solution or ointment. Further, the administration of the compound of the formula or composition of the invention may be in single dose or in multiple applications.

In some embodiments, the subject may be a mammal, preferably a human. In some embodiments, the compound of the invention is ML-A1-B or ML-A1-C. Either compound (i.e., ML-A1-B or ML-A1-C) may be administered to the subject in a dose of about 1 to 21,600 µg/Kg/day; preferably, 100 to 20,000, 100 to 15,000, 100 to 10,000, 100 to 5,000, 100 to 2,500, 100 to 1,000, 100 to 500, 500 to 20,000, 500 to 10,000, 500 to 5,000, 500 to 2,500 or 500 to 1,000µg/Kg/day

It will be appreciated that the dosage of the compound or composition of the invention will vary from patient to patient not only for the particular compound selected, the route of administration, and the ability of the compound to elicit a desired response in the patient, but also factors such as disease state or severity of the condition to be alleviated, age, sex, weight of the patient, the state of being of the patient, and the severity of the pathological condition being treated, concurrent medication or special diets then being followed by the patient, and other factors which those skilled in the art will recognize, with the appropriate dosage ultimately being at the discretion of the attendant physician. Dosage regimens may be adjusted to provide the desired response. Preferably, the compound of the present invention is administered at an amount of about 1 µg/kg/day to about 500 µg/kg/day, assuming the subject is about 60 kg in weight. More preferably, the compound of the present invention is administered at an amount of about 50 µg/kg/day to about 400 µg/kg/day; still more preferably, about 100 µg/kg/day to about 300 µg/kg/day; most preferably, about 160 µg/kg/day, and for a time such as 1 day, such that improved therapeutic response may be elicited in the subject. In some embodiments, the compound or composition of the invention is administered in single dosages. In other embodiments, the compound or composition of the invention is administered in multiple independent dosages.

The present invention will now be described more specifically with reference to the following embodiments, which are provided for the purpose of demonstration rather than limitation.

### Examples

### Materials and Methods

### Human cell isolation and cell culture

White blood cells of test subjects including healthy volunteers recruited from the Taiwan Blood Service Foundation (Taipei, Taiwan) and septic patients were obtained with written informed consents. Human monocytes were then isolated in accordance with procedures described previously (Chen IH et al., Cancer Immunol Immunother (2010) 59, 323-334). Briefly, peripheral blood mononuclear cells (PBMCs) were isolated using Ficoll-Paque Plus (GE Healthcare) gradient centrifugation. The monocytes were further purified by conducting CD14 selection using CD14 MACS microbeads (Miltenyi Biotec). The purity of monocytes confirmed using flow cytometry analysis was approximately 90%. Monocytes were incubated with endotoxin LPS (100 ng/ml) for 72 hours. At the indicated time point, the cells were harvested and the PD-L1 and HLA-DR molecules were analyzed using flow cytometry. Murine B16F10 cell lines maintained in Dulbecco's modified Eagle's medium (DMEM), 10% heat-inactivated fetal calf serum, 2mM L-glutamine, penicillin (100U/ml), and streptomycin (100 µg/ml) at 37C in a 5% CO₂ humidified atmosphere.

### Flow cytometry analysis

To analyze the surface phenotype of the monocytes, the cells were incubated for 30 minutes on ice in the dark with the following mAbs diluted in phosphate-buffered saline (PBS) containing 1% BSA: PD-L1-FITC, HLA-DR-PE, CD14-PerCP (BD Biosciences). Monocytes were gated based on their FSC/SSC properties. Fluorescence was detected using FACS Calibur, and data analysis was performed using FCS Express version 3 (De Novo Software).

### Patient inclusion criteria

21 patients with septic shock and 20 healthy volunteers in an observational study were enrolled in this study with written informed consent from each subject, and the study was approved by the Institutional Review Board of Mackay Memorial Hospital (Taipei, Taiwan). Septic shock was judged according to the criteria established at the American College of Chest Physicians/Society of Critical Care Medicine Conference. Exclusion criteria included the following: age < 20; leukemia; and receiving chemotherapy or immunosuppressive therapy. The following clinical and biological data were collected when patients were admitted to an intensive care unit: age; sex; acute physiology and chronic health evaluation (APACHE II) score (see Knaus WA et al., Crit Care Med. (1985) 13, 818-829); and sepsis-related organ failure assessment (SOFA) scores (see Vincent JL et al., Intensive Care Med. (1996) 22, 707-710).

The APACHE II scoring system is a severity-of-disease classification system which has been validated in the first 24 hours of admission of patients to an intensive care unit (ICU). The score is calculated from 12 physiological variables during the first 24 hours after admission and ranges from 0 to 71 points.

The SOFA scoring system, the sequential organ failure assessment score, is a patient's organ dysfunction and morbidity severity score during the patient's stay in an ICU. The score is calculated from 6 variables: 1. respiratory system; 2. cardiovascular systems; 3. hepatic systems; 4. coagulation systems; 5. renal systems; 6. neurological systems. Each variable is given a point value from 0 to 4 (normal to high degree of organ dysfunction). Total score ranges from 0 to 24.

### Cell viability assay

5 x 10³ of B16F10 cells were seeded into 96-well plates and treated with the following: DMSO control, various concentrations of naphthalimide derived compounds for 2 day. For the MTS assay (Promega), 40 ul of the MTS reagent was added into each well. Cells were incubated at 37 °C for 4hr. The absorbance was detected at OD490 nm.

### Compounds therapy in B16F10 lung metastasis model

C57BL/6 mice (6 to 8 weeks old) and NOD/SCID mice (6 to 8 weeks old) were purchased from the National Laboratory Animal Center (Taipei, Taiwan). All animal experiments were performed under specific pathogen-free conditions and in accordance with guidelines approved by the Animal Care and Usage Committee of Mackay memorial hospital (Taipei, Taiwan).

To generate lung metastases, mice were injected intravenously with 2x10⁵ B16F10 cells, a dose consistently yielding lung metastases in 100% of animals. 2mg/kg compounds and 5% DMSO vehicle control were given ip daily to test the effect of compounds on tumor metastatsis. The amount of tumor seeding was counted as total numbers of black nodules presented in the lungs under microscopy.

### In vivo toxic study

C57BL/6 mice were ip injected daily with 20 mg/kg of ML-A1-B, or ML-A1-C, or 5% DMSO vehicle control, and their body weight were measured on the indicated days.

### Compounds and anti-PD-1 antibody combination therapy in B16F10 lung metastasis model

C57BL/6 mice were injected iv with 2 x 10⁵ B16F10 cells on Day0. On Day1, mice were injected ip with either control hamster Ig (CTRL IgG) or hamster anti-mouse-PD-1 (J43) at 5 mg/kg every 3-4 days. For combination therapies, mice were daily injected with 2 mg/kg of ML-C19-A or ML-A1-C combined with anti-mouse PD-1 (5mg/kg) every 3-4 days. Long-term of survival of treated mice in each of the experimental groups were observed.

### Statistical analysis

All data were analyzed using Prism 6.0 (GraphPad) and expressed as mean ± standard error of mean (SEM) unless otherwise indicated. Comparisons between groups were performed using the Student *t* test. Correlations were determined using the Pearson's correlation coefficient. A probability value of *p* <0.05 was considered statistically significant.

### Example 1 Synthesis of the Compound of the Present Invention

### 1.1 Synthesis of the Compound ML-C19-B

ML-C19-B compound was prepared in accordance with the following scheme:

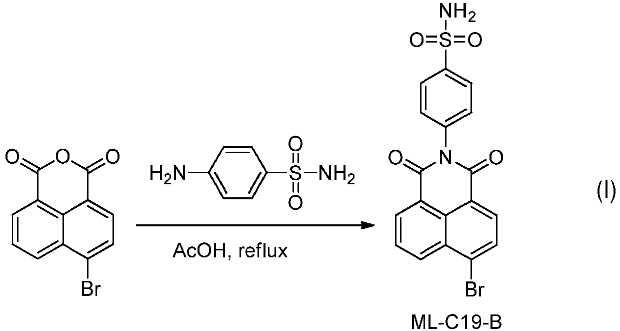

4-bromo-1,8,-naphthalic anhydride (1.0 mmol), sulfanilamide (0.1894 g, 1.1 mmol), and 5 mL of glacial acetic acid were thoroughly mixed and heated under reflux for 8 hours. After the reaction was completed, the mixture was cooled and the product was precipitated by the addition of cold water. The resulting solid was filtered and washed several times with cold water. The solid was recrystallized from 95% ethanol to yield the target molecule ML-C19-B.

Compound ML-C19-B: 4-(6-bromo-1,3-dioxo-1H-benzo[de]isoquinolin-2(3H)-yl)benzene-sulfonamide. MS electrospray (+ ion) 432.300 (MH+). ¹H NMR (600 MHz, DMSO - *d*₆) : ***δ*** 8.54 (d, *J* = 8.4 Hz, 2H), 8.32 (d, *J* = 8.4 Hz, 2H), 8.22 (d, *J* = 7.6 Hz, 2H), 8.01 (t, *J =* 13.2 Hz, 2H), 7.73 (d, *J* = 8.4 Hz, 1H), 7.62 (d, *J* = 7.8 Hz, 1H), 7.50 (s, NH). ¹³C NMR (150 MHz, DMSO - *d*₆) : ***δ*** 168.77, 162.9, 160.5, 143.9. 142.1, 138.7, 133.2, 132.6, 131.5, 130.9, 129.1, 129.3, 128.7, 127.3, 126.4, 119.8, 112.4.

### 1.2 Synthesis of the Compounds ML-A1-B and ML-A1-C

ML-A1-B and ML-A1-C compounds were prepared in accordance with the following scheme:

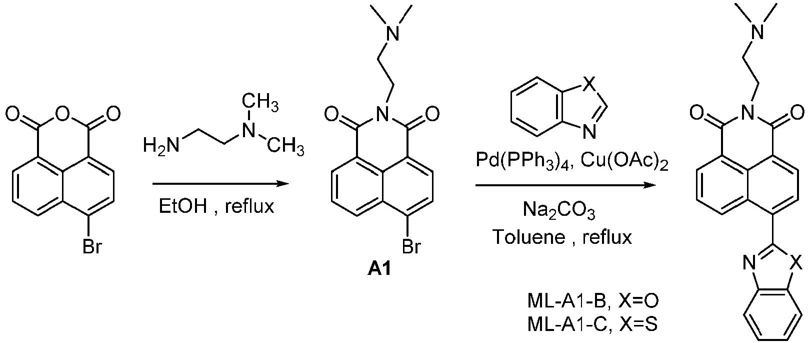

4-bromo-1,8-naphthalic anhydride (1.000 g, 3.610 mmol) and N,N-Dimethyl ethylene-diamine (0.3819 g, 4.3320 mmol) were dissolved in ethanol (20 mL) and subjected to heating under reflux for 8 hours. The reaction mixture was then cooled to room temperature, and the yellowish sediments were collected by filtration and dried overnight at room temperature under vacuum to produce Compound A1 (1.0250 g, 2.952 mmol).

Benzoazoles (2.8816 mmol), compound A1 (0.500 g, 1.4408 mmol), Pd(PPh₃)₄ (14.4 mg, 1.0 mol%), Cu(OAc)₂ . H₂O (57.5 mg, 20 mol%), and Na₂CO₃ (0.3050 g, 2.880 mmol) were dissolved in toluene (10 mL) and subjected to heating under reflux for 10 hours. This mixture was then cooled to room temperature, filtered and the filtrate was evaporated under reduced pressure. The residue was purified by column chromatography (silica gel, Hexane/EtOAc as eluent) to give arylated benzoxazole ML-A1-B and ML-A1-C.

Compound ML-A1-B: 6-(Benzo[d]oxazol-2-yl)-2-(2-(dimethylamino)ethyl)-1H-benzo[de]isoquinoline-1,3(2H)-dione. MS electrospray (+ ion) 386.500 (MH+). 1H NMR (600 MHz, DMSO - d6) : δ 9.86 (d, J = 8.4 Hz, 1H), 8.72 (d, J = 7.8 Hz, 1H), 8.65 (d, J = 8.4 Hz, 1H), 8.62 (d, J = 7.2 Hz, 1H), 8.09 (t, J = 16.2 Hz, 1H), 7.99 (d, J = 7.8 Hz, 7.91 (d, J = 7.8 Hz, 1H), 7.56 (t, J = 15.6 Hz, 1H), 7.51 (t, J = 15.0 Hz, 1H), 4.17 (t, J = 13.8 Hz, 2H), 2.57 (t, J = 10.8 Hz, 2H), 2.23 (s, 6H). 13C NMR (150 MHz, DMSO - d6) : δ 163.2, 162.8, 160.4, 149.7, 141.5, 132.4, 131.2, 130.0, 129.5, 128.9, 128.3, 128.1, 127.7, 126.8, 125.3, 124.6, 122.7, 120.5, 111.2, 59.4, 45.4, 37.8. HRMS (ESI) Calculated for C23H20N3O3 ([M+H]⁺) 386.4153, Found: 386.1499.

Compound ML-A1-C: 6-(Benzo[d]thiazol-2-yl)-2-(2-(dimethylamino)ethyl)-1H-benzo[de]isoquinoline-1,3(2H)-dione. MS electrospray (+ ion) 402.400 (MH+). 1H NMR (600 MHz, CD3OD) : δ 9.41 (d, J = 8.4 Hz, 1H), 8.64 (t, J = 13.2 Hz, 2H), 8.25 (d, J = 7.2 Hz, 1H), 8.15 (d, J = 8.4 Hz, 1H), 8.08 (d, J = 7.8 Hz, 1H), 7.91 (t, J = 15.6 Hz, 1H), 7.61 (t, J = 15.0 Hz, 1H), 7.53 (t, J = 15.0 Hz, 1H), 4.55 (t, J = 11.4 Hz, 2H), 3.57 (t, J = 10.8 Hz, 2H), 3.05 (s, 6H). 13C NMR (150 MHz, CD3OD) : δ 167.1, 166.0, 165.6, 155.5, 137.8, 136.9, 134.8, 133.1, 131.8, 131.2, 130.4, 130.3, 129.6, 128.3, 127.7, 125.1, 124.9, 123.8, 123.11, 57.7, 44.5, 36.9. HRMS (ESI) Calculated for C23H19N3O2S ([M+H]+) 402.4809, Found: 403.1404.

### 1.3 Synthesis of the Compounds ML-C19-PH2, ML-C19-PH3, ML-C19-PH4 and ML-C19-PH6

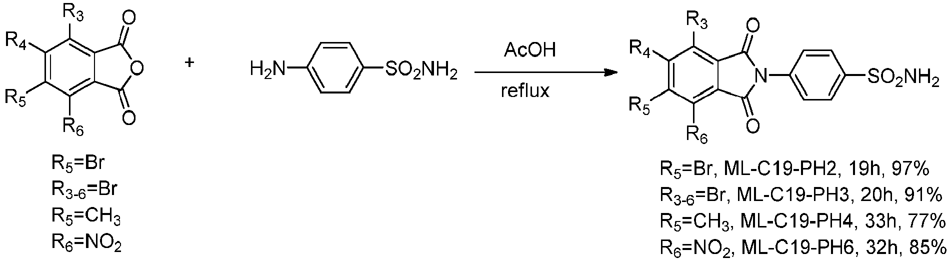

The substituted phthalic anhydride and sulfanilamide were heated with excess amount of AcOH under reflux condition for 20h. The solid was then filtrated and washed with EtOH.
**ML-C19-PH2:** ¹H NMR (300 MHz, DMSO-*d*₆) δ: 8.20 (d, *J*=1.6 Hz, 1H), 8.10 (dd, *J* = 8.0, 6.4 Hz, 1H), 7.99-7.67 (m, 3H), 7.66 (d, *J* = 7.1 Hz, 2H), 7.47 (s, 2H). HRMS calculated for C₁₄H₉BrN₂O₄S 379.9466. Found: 379.9465.
**ML-C19-PH3:** ¹H NMR (300 MHz, DMSO-*d*₆) δ: 7.99 (d, *J*=8.6 Hz, 2H), 7.64 (d, *J* = 8.6 Hz, 2H), 7.49 (s, 2H).
**ML-C19-PH4:** ¹H NMR (300 MHz, DMSO-*d*₆) δ: 7.96 (dd, *J*=6.6, 1.5 Hz, 2H), 7.88 (d, *J* = 7.6 Hz, 1H), 7.82 (s, 1H), 7.70 (d, *J* = 10.2 Hz, 1H), 7.65 (dd, *J* = 7.0, 1.7 Hz, 2H), 7.46 (s, 2H), 2.53 (s, 3H). HRMS calculated for C₁₅H₁₂N₂O₄S 316.0518. Found: 316.0523.
**ML-C19-PH6:** HRMS calculated for C₁₄H₉N₃O₆S 347.0212. Found: 347.0218.

### Example 2 Levels of PD-L1 and HLA-DR are Respectively Up- and Downregulated in Septic Shock Patients

To identify the protein target responsible for sepsis or septic shock, a group of septic shock patient and healthy volunteers were recruited in the present study from April 2013 to April 2014 based on the inclusion criteria set forth in the "Materials and Methods" section, and blood samples were taken within 6 hours upon admission, and cell-surface PD-L1 and HLA-DR expression in CD14+ monocytes were measured via flow cytometry analysis.

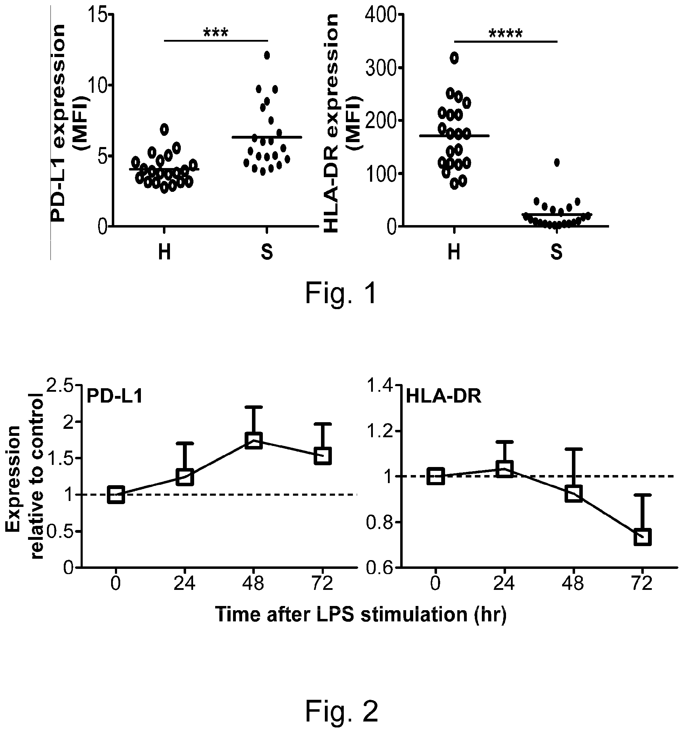

Respective clinical characteristics of the septic shock patients and the healthy volunteers are summarized in Table 1; and the expression levels of PD-L1 and HLA-DR on monocytes are depicted in FIG 1.

**Table 1 Clinical characteristics of septic shock patients and healthy volunteers**

| | Patients with septic shock (n = 21) | Healthy volunteers (n = 20) |
|---|---|---|
| Age | 61.8±3.3 | 64.4±0.8 |
| Sex ratio, male/female | 10/11 | 12/8 |
| APACHE II score, median [range] | 25 [11-35] | NA |
| SOFA score, median [range] | 11 [6-16] | NA |

According to clinical characteristics summarized in Table 1, septic shock patients in the present study have a median SOFA score of 11, suggesting multiple organ dysfunction, and a median APACHE II score of 25, suggesting patients were in the early stage of severe sepsis.

According to the results illustrated in Fig. 1, the enrolled septic shock patients exhibited significantly elevated levels of PD-L1 (septic shock: 6.3 ± 0.5 vs normal: 4.1 ± 0.2, P < 0.001). By contrast, the levels of HLA-DR in patients with septic shock were significantly lower as compared with those of healthy volunteers (septic shock: 22.4 ± 5.9 vs normal: 171.3 ± 14, P<0.0001). These findings led to the hypothesis that PD-L1, HLA-DR or both may serve as the protein target(s) responsible for immunoparalysis in septic shock patients.

### Example 3 Therapeutic Effects of the Compound

### 3.1 Lipopolysaccharide (LPS)-primed monocyte model system

In this example, an LPS-primed monocyte model was established and then used to evaluate the function of the compound of example 1. Specifically, monocytes isolated from healthy subjects according to procedures described in the "Materials and methods" section were incubated with LPS (100 ng/mL) for 72 hours, then respective levels of PD-L1 and HLA-DR were determined. Results are illustrated in Fig. 2.

As depicted in Fig. 2, after LPS treatment, the levels of PD-L1 on monocytes increased along with time, whereas a decrease in the levels of HLA-DR was observed at the same time; such observation is similar to the phenomenon seen in a septic shock patient of Example 2. Thus, LPS-primed monocyte is a suitable cell model system for screening a potential candidate compound(s) for the treatment of sepsis or septic shock.

### 3.2 Effects of the compounds of example 1 on the levels of PD-L1 and HLA-DR in LPS-primed monocyte model of section 3.1

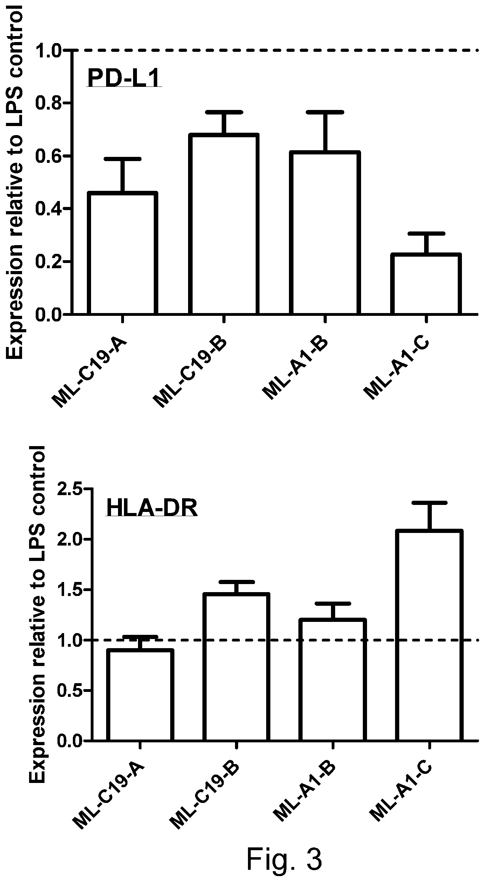

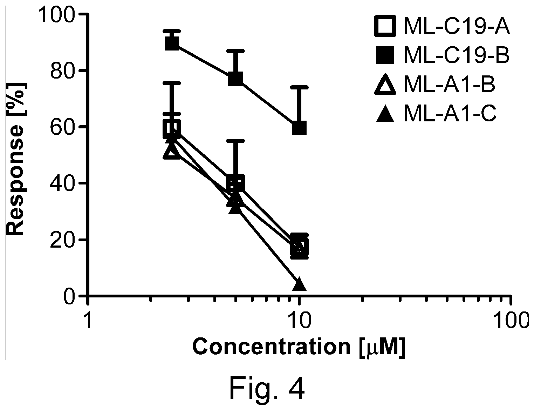

The effects of the compounds of example 1 were tested in the LPS-primed monocyte model of example 3.1, in which various concentrations of ML-C19-A, ML-C19-B, ML-A1-B, and ML-A1-C were incubated with LPS-primed monocytes of example 3.1; results are illustrated in Figs. 3 and 4.

As depicted in Fig. 3, the compounds ML-C19-A, ML-A1-B, and ML-A1-C (respectively at 10 µM) were effective in reversing the LPS-induced increase in PD-L1; with the expression level of PD-L1 in LPS-primed monocytes reduced to about 37.5%, 9.4%, and 50.8% of that of the control (dashed line). As to ML-C19-B, it appeared to be less potent than ML-C19-A, merely 20% reduction in PD-L1 level was observed, as compared with that of the control. In contrast, ML-C19-B, ML-A1-B, and ML-A1-C, were capable of altering the levels of HLA-DR expression. The dose dependency of ML-C19-A ML-C19-B, ML-A1-B, or ML-A1-C on the level of PD-L1 is illustrated in Fig. 4.

Taken together, the results suggest that compounds ML-C19-A, ML-C19-B, ML-A1-B, and ML-A1-C may serve as potential lead compounds for manufacturing a medicament for treating sepsis or septic shock, for each compound is capable of reversing the increase in PD-L1 observed in a septic shock patient.

### Example 4 In vitro effect on PDL1 expression of B16F10

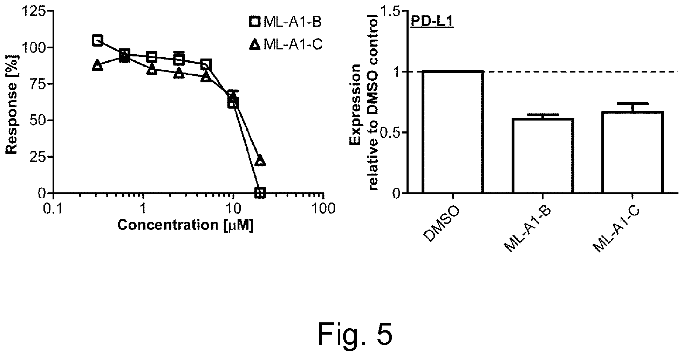

To evaluate the effect of ML-A1-B and ML-A1-C on PD-L1 expression of on B16F10 melanoma cells, IFN-γ stimulated B16F10 cells were treated with various concentrations of ML-A1-B or ML-A1-C compounds for 1 day. As shown in Fig. 5, the up-regulation of PD-L1 by IFN-γ can be blocked by administration of ML-A1-B and ML-A1-C compounds. ML-A1-B and ML-A1-C significantly suppresses PD-L1 on IFN-γ stimulated murine B16F10 melanoma cell line. The inhibition increased sharply at concentration higher than 10 µM, partly because of cell toxicity effect by both compounds. Together, these results suggest that ML-A1-B and ML-A1-C play a crucial role in the inhibition of PD-L1 expression on IFN-γ stimulated B16F10 cancer cells and LPS-primed monocytes.

### Example 5 In vitro cytotoxic effect

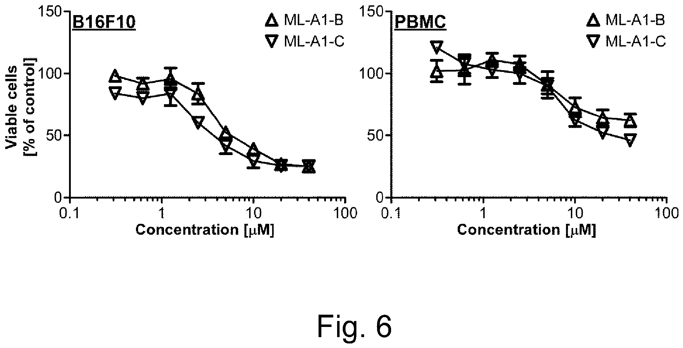

The cytotoxicity of various concentrations of ML-A1-B and ML-A1-C against murine B16F10 melanoma cells and human PBMCs were examined by MTS assay. As shown in Fig. 6, ML-A1-B and ML-A1-C significantly increases cell damage (MTS assay) to melanoma cell line (B16F10) with a dose-dependent response (IC₅₀ of ML-A1-C: 7.8 ± 0.05 uM; IC₅₀ of ML-A1-C: 4.5 ± 0.06 uM). In contrast, human PMBCs are relatively resistant to ML-A1-B and ML-A1-C treatment (IC₅₀ of ML-A1-C: 48.3 + 0.14 uM; IC₅₀ of ML-A1-C: 25.3 + 0.08 uM). Therapeutic index by MTS assay is about 5.62.

### Example 6 In vivo effects on B16F10 lung metastatic models

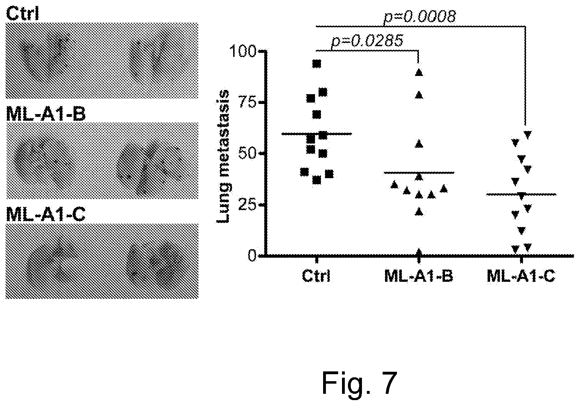

To examine the therapeutic effect of ML-A1-B and ML-A1-C against pre-established B16F10 lung metastases, mice were iv injected with 2 x 10⁵ B16F10 tumor cells and treated with, per mouse, 2 mg/kg of ML-A1-B, ML-A1-C, or with DMSO vehicle control. The number of lung nodules in each individual mouse in each group is shown in Fig. 7. Mice treated with ML-A1-B showed a reduction in the number of lung metastases (mean 41; range 2-90) as compared with mice receiving the DMSO vehicle control (mean 60; range 37-94). In contrast, the 2 mg/kg of ML-A1-C had a marked effect in reducing metastasis to the lung, with a mean of 30 lung nodules (range 3-59).

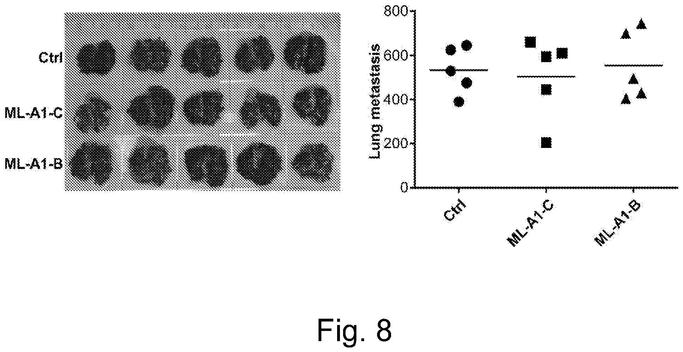

To answer the question of whether ML-A1-B and ML-A1-C are still capable of directly inhibiting tumor growth in the absence of an adaptive immune response, we injected 2 x 10⁵ B16F10 tumor cells and treated them with, per mouse, 2 mg/kg of ML-A1-B, ML-A1-C, or with DMSO vehicle control in NOD/SCID mice. As shown in Fig 8, treatment with ML-A1-B or ML-A1-C failed to inhibit lung metastasis in immune deficient NOD/SCID mice, suggesting that anti-tumor activity of ML-A1-B and ML-A1-C is through modulating the immune system against tumor growth, not through directly killing tumors.

It can be concluded that ML-A1-B and ML-A1-C may provide a novel therapeutic cancer drug to treat PD-L1 associated cancer by simultaneously modulating PD-L1 expression on cancer cells and inhibiting tumor growth. ML-A1-B and ML-A1-C would be the first-in-class small molecule for cancer immunotherapy, if it can succeed in future trials. We anticipate that ML-A1-B and ML-A1-C may not only inhibit tumor growth but also contribute to amelioration of the immune deficiency observed in cancer patients. If the results demonstrated here hold true in clinical, ML-A1-B and ML-A1-C treatment in patients with cancer may help to achieve optimal outcomes and improved survival. Compared with clinically available therapeutic antibodies (anti-PD-1, CTLA-4), the cost of treatment and manufacturing requirements are certainly lower than those of antibodies.

### Example 7 In vivo toxic assay

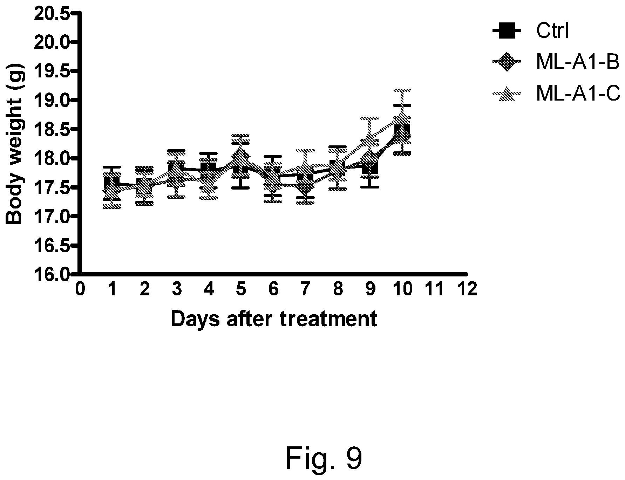

To investigate toxicity of ML-A1-B and ML-A1-C, mice were injected ip daily with 20 mg/kg of ML-A1-B or ML-A1-C or 5% DMSO vehicle control. As shown in Fig. 9, the compounds of the invention, ML-A1-B and ML-A1-C, do not significantly reduce the body weight of the mice and no visible sign of toxicity were observed in high dose of ML-A1-B and ML-A1-C treated mice.

### Example 8 Combination of anti-PD-1 with compounds ML-C19-A and M-A1-C

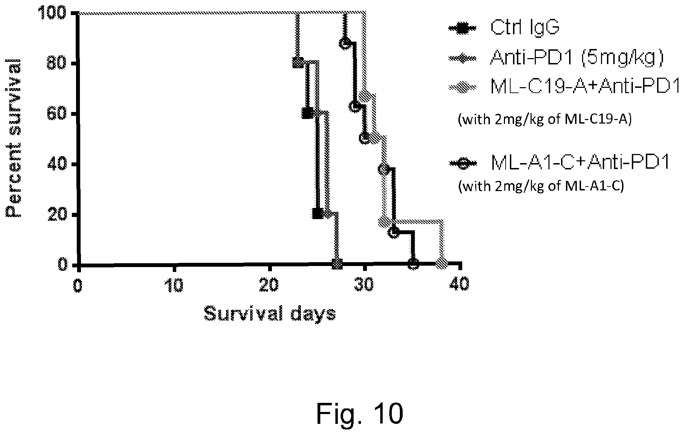

To examine whether combination of ML-C19-A or ML-A1-C with anti-PD-1 antibody would prolong mice survival in B16F10 lung metastasis model, mice were injected ip with compounds and anti-PD-1 antibody. As shown in Fig. 10, mice receiving concurrent treatments of compounds (2mg/kg) and anti-PD-1 (5mg/kg) demonstrated an increase in overall survival.

It will be understood that the above description of embodiments is given by way of example only and that various modifications may be made by those with ordinary skill in the art. The above specification, examples and data provide a complete description of the structure and use of exemplary embodiments of the invention. Although various embodiments of the invention have been described above with a certain degree of particularity, or with reference to one or more individual embodiments, those with ordinary skill in the art could make numerous alterations to the disclosed embodiments without departing from the spirit or scope of this invention.

## Claims

1. A compound having the following Formula (I), 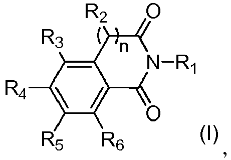 wherein the compound is selected from the group consisted of the following:
| Compound Name | n | R₁ | R₂ | R₃ | R₄ | R₅ | R₆ |
|---|---|---|---|---|---|---|---|
| ML-A1-B | 1 | -CH₂CH₂N(CH₃)₂ | =CH-CH=CH- | | 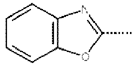 | H | H |
or a tautomer, stereoisomer or enantiomer thereof, or a solvate, or a pharmaceutically acceptable salt thereof.

2. A pharmaceutical composition for use in treating or preventing a PD-L1 associated cancer in a subject by inhibiting PD-L1, wherein the pharmaceutical composition comprises the compound having the following formula (I): 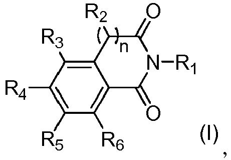 wherein the compound is selected from the group consisted of the following:
| Compound Name | n | R₁ | R₂ | R₃ | R₄ | R₅ | R₆ |
|---|---|---|---|---|---|---|---|
| ML-A1-B | 1 | -CH₂CH₂N(CH₃)₂ | =CH-CH=CH- | | 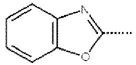 | H | H |
| ML-A1-C | 1 | -CH₂CH₂N(CH₃)₂ | =CH-CH=CH- | | 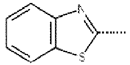 | H | H |
| ML-C19-A | 1 | 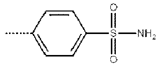 | =CH-CH=CH- | | H | H | H |
or a tautomer, stereoisomer or enantiomer thereof, or a solvate, or a pharmaceutically acceptable salt thereof.

3. The pharmaceutical composition for use according to Claim 2, further comprising a second active agent, wherein the second active agent is an anticancer agent or an immune checkpoint inhibitor.

4. The pharmaceutical composition for use according to Claim 2, wherein the immune checkpoint inhibitor is an anti-PD-Ll antibody, an anti-PD-1 antibody, or an anti-CTLA-4 antibody.

5. The pharmaceutical composition for use according to Claim 2, wherein the immune checkpoint inhibitor is ipilimumab, lambrolizumab, MPDL3280A, MEDI4736, or avelumab.

6. The pharmaceutical composition for use according to Claim 2, wherein the anticancer agent is an antimetabolite, antimicrotubule agent, alkylating agent, platinum agent, anthracycline, antitumor antibiotic, topoisomerase inhibitor, purine antagonist or pyrimidine antagonist, cell maturing agent, DNA repair enzyme inhibitor, histone deacetylase inhibitor, cytotoxic agent, hormone, anti-cancer monoclonal antibody, immuno-modulator, Bcr-Abl kinase inhibitor, or a hormone agonist or antagonist.

## Patentansprüche

1. Verbindung mit der folgenden Formel (I): 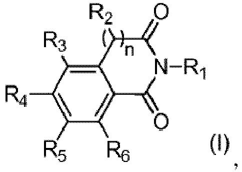 wobei die Verbindung ausgewählt ist aus der Gruppe, bestehend aus Folgendem:
| Name der Verbindung | n | R₁ | R₂ | R₃ | R₄ | R₅ | R₆ |
|---|---|---|---|---|---|---|---|
| ML-A1-B | 1 | -CH₂CH₂N(CH₃)₂ | =CH-CH=CH- | | 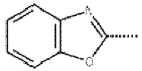 | H | H |
oder einem Tautomer, Stereoisomer oder Enantiomer davon, oder einem Solvat oder einem pharmazeutisch annehmbaren Salz davon.

2. Pharmazeutische Zusammensetzung zur Verwendung bei der Behandlung oder Verhütung einer PD-L1-assoziierten Krebserkrankung in einem Subjekt durch die Inhibition von PD-L1, wobei die pharmazeutische Zusammensetzung die Verbindung mit der folgenden Formel (I) umfasst: 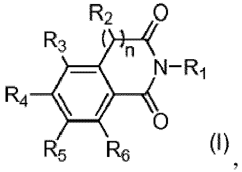 wobei die Verbindung ausgewählt ist aus der Gruppe, bestehend aus Folgendem:
| Name der Verbindung | n | R₁ | R₂ | R₃ | R₄ | R₅ | R₆ |
|---|---|---|---|---|---|---|---|
| ML-A1-B | 1 | -CH₂CH₂N(CH₃)₂ | =CH-CH=CH- | | 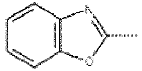 | H | H |
| ML-A1-C | 1 | -CH₂CH₂N(CH₃)₂ | =CH-CH=CH- | | 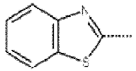 | H | H |
| ML-C19-A | 1 | 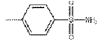 | =CH-CH=CH- | | H | H | H |
oder einem Tautomer, Stereoisomer oder Enantiomer davon, oder einem Solvat oder einem pharmazeutisch annehmbaren Salz davon.

3. Pharmazeutische Zusammensetzung nach Anspruch 2, weiter umfassend einen zweiten Wirkstoff, wobei der zweite Wirkstoff ein Antikrebsmittel oder ein Immuncheckpoint-Inhibitor ist.

4. Pharmazeutische Zusammensetzung zur Verwendung nach Anspruch 2, wobei der Immuncheckpoint-Inhibitor ein Anti-PD-L1-Antikörper, ein Anti-PD-1-Antikörper oder ein Anti-CTLA-4-Antikörper ist.

5. Pharmazeutische Zusammensetzung zur Verwendung nach Anspruch 2, wobei der Immuncheckpoint-Inhibitor Ipilimumab, Lambrolizumab, MPDL3280A, MEDI4736 oder Avelumab ist.

6. Pharmazeutische Zusammensetzung zur Verwendung nach Anspruch 2, wobei das Antikrebsmittel ein Antimetabolit, Antimikrotubulmittel, Alkylierungsmittel, platinhaltiges Mittel, Anthracyclin, Antitumor-Antibiotikum, Topoisomeraseinhibitor, Purinantagonist oder Pyrimidinantagonist, Zellenreifungsmittel, DNA-Reparaturenzym-Innhibitor, Histon-Deacetylase-Inhibitor, cytotoxisches Mittel, Hormon, monoklonaler Antikrebs-Antikörper, Immunmodulator Bcr-AbL-Kinase-Inhibitor oder Hormon-Agonist oder -Antagonist ist.

## Revendications

1. Composé de formule (I) suivante, 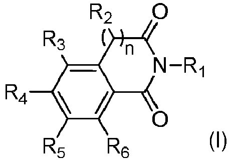 lequel composé est choisi dans le groupe constitué par les suivants :
| Nom du composé | n | R₁ | R₂ | R₃ | R₄ | R₅ | R₆ |
|---|---|---|---|---|---|---|---|
| ML-A1-B | 1 | -CH₂CH₂N(CH₃)₂ | =CH-CH=CH- | | 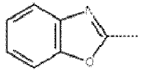 | H | H |
ou un tautomère, stéréoisomère ou énantiomère de celui-ci, ou un solvate, ou un sel pharmaceutiquement acceptable de celui-ci.

2. Composition pharmaceutique pour une utilisation dans le traitement ou la prévention d'un cancer associé à PD-L1 chez un sujet par inhibition de PD-L1, laquelle composition pharmaceutique comprend le composé de formule (I) suivante : 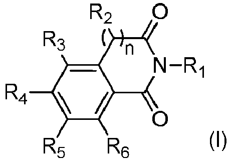 dans laquelle le composé est choisi dans le groupe constitué par les suivants :
| Nom du composé | n | R₁ | R₂ | R₃ | R₄ | R₅ | R₆ |
|---|---|---|---|---|---|---|---|
| ML-A1-B | 1 | -CH₂CH₂N(CH₃)₂ | =CH-CH=CH- | | 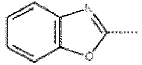 | H | H |
| ML-A1-C | 1 | -CH₂CH₂N(CH₃)₂ | =CH-CH=CH- | | 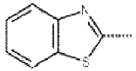 | H | H |
| ML-C19-A | 1 | 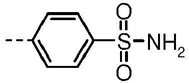 | =CH-CH=CH- | | H | H | H |
ou un tautomère, stéréoisomère ou énantiomère de celui-ci, ou un solvate, ou un sel pharmaceutiquement acceptable de celui-ci.

3. Composition pharmaceutique pour une utilisation selon la revendication 2, comprenant en outre un deuxième principe actif, dans laquelle le deuxième principe actif est un agent anticancéreux ou un inhibiteur de point de contrôle immunitaire.

4. Composition pharmaceutique pour une utilisation selon la revendication 2, dans laquelle l'inhibiteur de point de contrôle immunitaire est un anticorps anti-PD-L1, un anticorps anti-PD-1, ou un anticorps anti-CTLA-4.

5. Composition pharmaceutique pour une utilisation selon la revendication 2, dans laquelle l'inhibiteur de point de contrôle immunitaire est l'ipilimumab, le lambrolizumab, MPDL3280A, MEDI4736, ou l'avélumab.

6. Composition pharmaceutique pour une utilisation selon la revendication 2, dans laquelle l'agent anticancéreux est un antimétabolite, un agent antimicrotubule, un agent d'alkylation, un agent au platine, une anthracycline, un antibiotique antitumoral, un inhibiteur de topoisomérase, un antagoniste de purine ou antagoniste de pyrimidine, un agent de maturation cellulaire, un inhibiteur d'enzyme de réparation de l'ADN, un inhibiteur d'histone désacétylase, un agent cytotoxique, une hormone, un anticorps monoclonal anticancéreux, un immunomodulateur, un inhibiteur de kinase Bcr-Abl, ou un agoniste ou antagoniste d'hormone.
